(19) Europäisches Patentamt
European Patent Office
Office européen des brevets

(11) **EP 1 873 514 A1**

(12) **EUROPEAN PATENT APPLICATION**
published in accordance with Art. 153(4) EPC

(43) Date of publication:
**02.01.2008 Bulletin 2008/01**

(21) Application number: **06745396.9**

(22) Date of filing: **12.04.2006**

(51) Int Cl.:
***G01N 21/64*** *(2006.01)*

(86) International application number:
**PCT/JP2006/307743**

(87) International publication number:
**WO 2006/115059 (02.11.2006 Gazette 2006/44)**

(84) Designated Contracting States:
**DE FR GB**

(30) Priority: **22.04.2005 JP 2005124571**

(71) Applicant: **Sony Corporation**
**Minato-ku**
**Tokyo (JP)**

(72) Inventors:
- **OHTO, Yasunori c/o SONY-KIHARA RESEARCH CENTER INC**
**Tokyo 1410022 (JP)**
- **TSUJIMOTO, Atsumi c/o SONY CORPORATION**
**Shinagawa-ku, Tokyo 1410001 (JP)**

(74) Representative: **Horner, David Richard**
**D Young & Co**
**120 Holborn**
**London EC1N 2DY (GB)**

(54) **BIOLOGICAL INFORMATION PROCESSING UNIT AND METHOD, PROGRAM AND RECORDING MEDIUM**

(57) The present invention relates to a biological-information processing apparatus and a method, which are capable of measuring a hybridization of a DNA chip without making use of a complex measurement configuration, at a low cost and with a high degree of accuracy, as well as relates to a program and a recording medium. An area enclosed by a spot boundary 461 is divided into spot internal areas 522 each having debris 463 and spot internal areas 521 each having no debris 463. For each of the spot internal areas 521 and 522, a flag f is set in accordance with the reliability of each of the spot internal areas 521 and 522 respectively in order to prevent data of a spot internal area having low reliability from being used. The present invention can be applied to an apparatus for measuring the fluorescence intensity of the DNA chip.

FIG.30

**Description**

[Technical Field]

The present invention relates to a biological-information processing apparatus, a biological-information processing method, a biological-information processing program and a program recording medium. More particularly, the present invention relates to a biological-information processing apparatus, a biological-information processing method, a biological-information processing program and a program recording medium, which are capable of measuring the state of a biological junction between biological substances without making use of a complex measurement configuration, at a low cost and with a high degree of accuracy.

[Background Art]

Practical applications of DNA (deoxyribonucleic acid) chips or DNA micro-arrays have been carried forward in recent years. The DNA chips or DNA micro-arrays will hereinafter be collectively referred to simply as "DNA chips" unless the DNA chips and the DNA micro-arrays need to be distinguished from each other. A DNA chip carries a number of various DNA oligonucleotide strands integrated and immobilized as detection nucleic acids on the surface of a substrate. By detecting a hybridization undergone by a probe immobilized on a spot on the substrate surface in conjunction with a target in samples collected from cells or the like making use of the DNA chip, a gene expression in the collected cells can be comprehensively analyzed.

With improvements of a hybridization detection technology in gene expression analyses carried out by using a DNA chip, it becomes not only possible to detect existence or non-existence of a gene expression, but also possible to quantitatively measure the quantity of the gene expression. For example, by quantitatively measuring the fluorescence intensity in a process to detect a hybridization, a technology for obtaining a quantitative numerical value showing a gene expression has been successfully put to practical use.

If a foreign substance exists on a DNA chip, it becomes difficult to accurately measure the fluorescence intensity required in a process to detect a hybridization undergone by a probe in conjunction with a target. In order to solve this problem, there has been proposed a method for eliminating the image of a foreign substance from the image of fluorescence intensities as disclosed in a document (such as following Patent Document 1).

[Patent Document 1]

Japanese Patent Laid-Open No. 2002-257730

[Disclosure of Invention]

[Problems to be Solved by the Invention]

In accordance with the method disclosed in Patent document 1, there are prepared a light beam having a first wavelength exciting a fluorescent substance and generating fluorescence and a light beam having a second wavelength exciting no fluorescent substance and generating no fluorescence. The light beam having the first wavelength is radiated to a DNA chip in order to obtain a first image (which is an image including portions completing a hybridization and a foreign substance) whereas the light beam having the second wavelength is radiated to the DNA chip in order to obtain a second image (which is the image of the foreign substance only). Then, the second image is eliminated from the first image.

However, an apparatus based on such a principle is required to employ light sources for generating light beams having two different wavelengths. Thus, the apparatus raises a problem of a complicated apparatus configuration and a problem of a high apparatus cost.

Addressing the problems described above, the present invention allows accurate measurements to be carried out without using an apparatus having a complicated configuration and a high price.

[Means for Solving the Problems]

In accordance with an aspect of the present invention, there is provided a biological-information processing apparatus for measuring a bioreaction undergone by a first biological substance immobilized in a reaction area provided on a substrate in conjunction with a second biological substance biologically reacting to the first biological substance. The biological-information processing apparatus includes:

inputting means for inputting an image of the reaction area; and
computation means for computing a reactivity representing the state of the bioreaction undergone by the first biological substance in conjunction with the second biological substance in the reaction area and a bioreaction reliability representing the reliability of the bioreaction on the basis of the image input by the inputting means.

As described above, the computation means employed in the biological-information processing apparatus according to the aspect of the present invention computes a reactivity representing the state of the bioreaction undergone by the first biological substance in conjunction with the second biological substance in the reaction area and a bioreaction reliability representing the reliability of the bioreaction on the basis of the image input by the inputting means.

The biological-information processing apparatus can also be further provided with setting means for setting a flag indicating whether or not the computed reactivity can be utilized.

The setting means can be made capable of setting the flag on the basis of the reliability, the number of sub-areas or information specified by the user. The sub-areas are partial areas obtained as a result of dividing the reactive area. The sub-areas include partial areas including an unnecessary substance and partial areas each including no unnecessary substance.

Thus, the biological-information processing apparatus can also be further provided with division means for dividing the reaction area into aforementioned partial areas each including an unnecessary substance and aforementioned partial areas each including no unnecessary substance. In this case, the computation means computes a reactivity and a reliability for each of the partial areas.

Partial-areas each included in one of a plurality of reaction areas used in measurements carried out under the same condition are combined to form a combination across the reaction areas. Then, for each combination, the computation means computes a combination reactivity representing the reactivity of the combination and a combination reliability representing the reliability of the combination, and the combination reactivity of a combination having the highest combination reliability among combinations is taken as the reactivity of the reaction areas.

A first biological substance and a second biological substance can be genes having mutually complementary base sequences or can each be a substance derived from the gene whereas a reactivity can be hybridization information of the first biological substance and the second biological substance.

The hybridization information can be information that can be determined unambiguously on the basis of a function from a fluorescence intensity of fluorescence obtained as a result of a hybridization undergone by the first biological substance in conjunction with the second biological substance.

The reliability can be information proportional to the reciprocal of the variance of points each included in a range as a point representing a fluorescence intensity obtained as a result of a hybridization undergone by the first biological substance in conjunction with the second biological substance in a first measurement and representing a fluorescence intensity obtained as a result of a hybridization undergone by the first biological substance in conjunction with the second biological substance in a second measurement.

[Effects of the Invention]

In accordance with the present invention, the state of a bioreaction can be measured. In particular, the measurement can be made at a low cost with a high degree of accuracy without making use of a complex measurement configuration.

[Brief Description of the Drawings]

[Fig. 1]

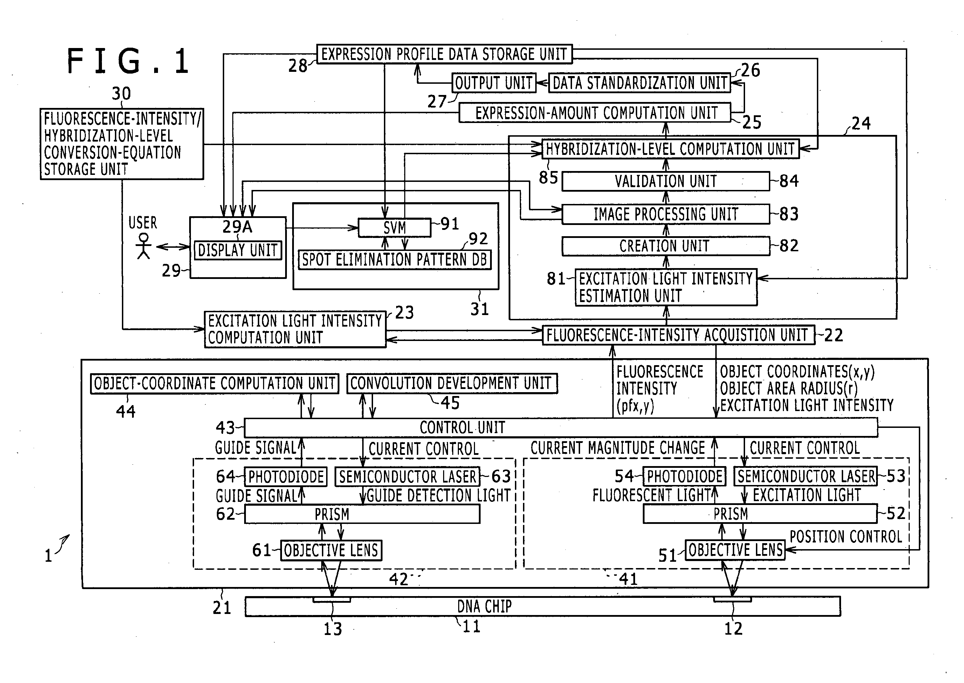

Fig. 1 is a block diagram showing a typical configuration of a biological-information processing apparatus according to an embodiment of the present invention.
[Fig. 2]

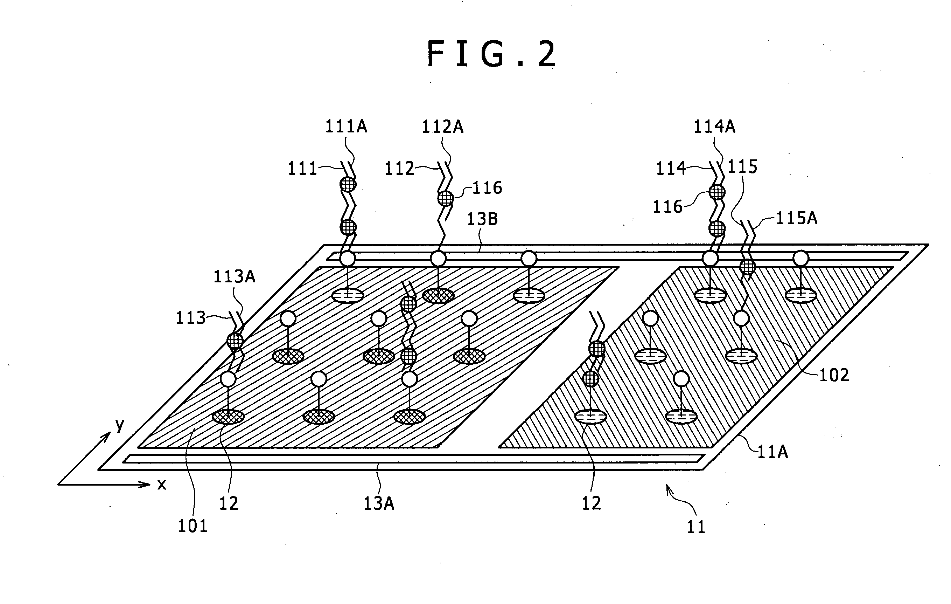

Fig. 2 is a perspective diagram showing a typical configuration of a DNA chip.
[Fig. 3]

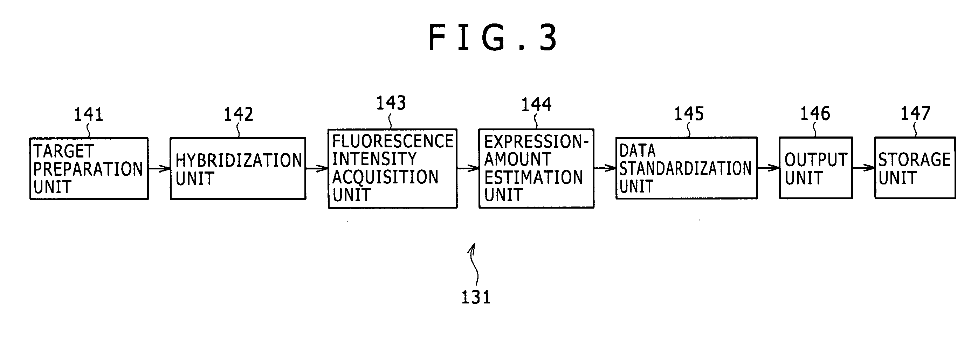

Fig. 3 is a block diagram showing a typical configuration of an experimental-process processing apparatus.
[Fig. 4]

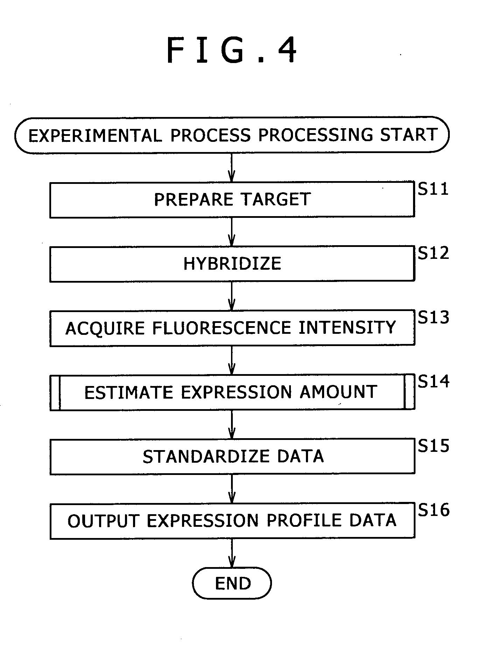

Fig. 4 shows a flowchart to be referred to in explanation of processing of an experimental process.
[Fig. 5]

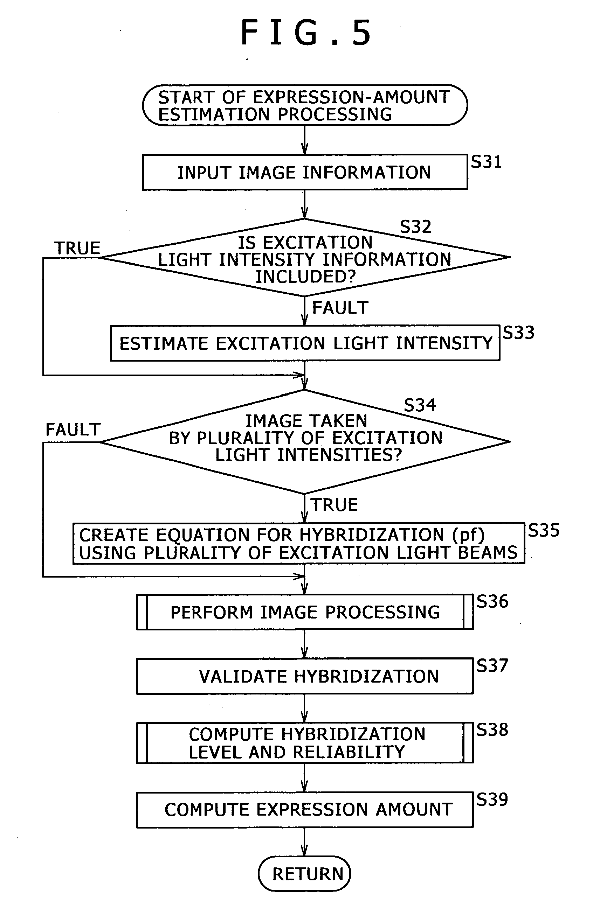

Fig. 5 shows a flowchart to be referred to in explanation of processing to estimate an expression amount.
[Fig. 6]

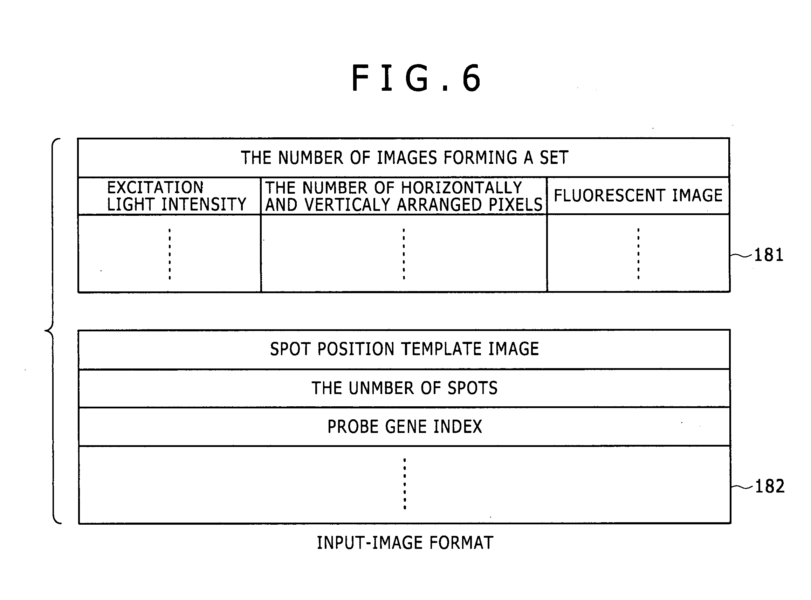

Fig. 6 is a diagram showing a typical format of an input image.
[Fig. 7]

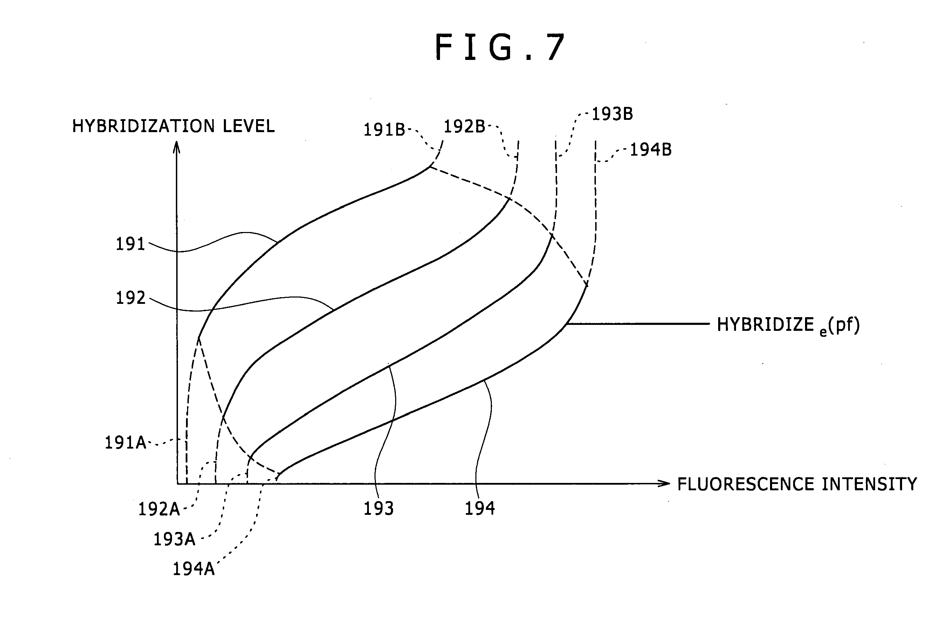

Fig. 7 is a diagram showing relations between a fluorescence intensity and a hybridization level.
[Fig. 8]

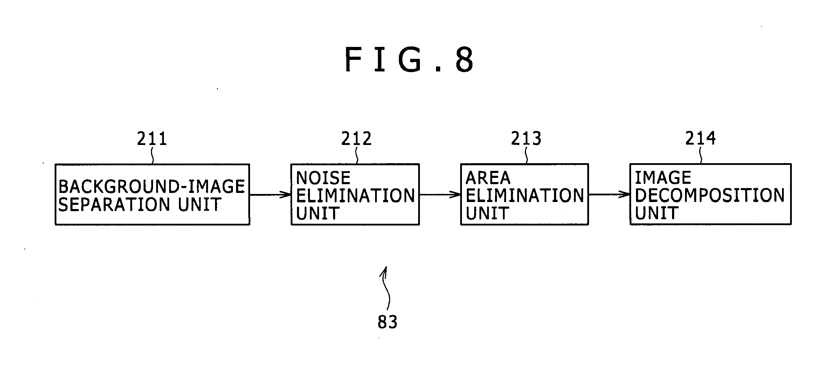

Fig. 8 is a block diagram showing a typical configuration of an image processing apparatus.
[Fig. 9]

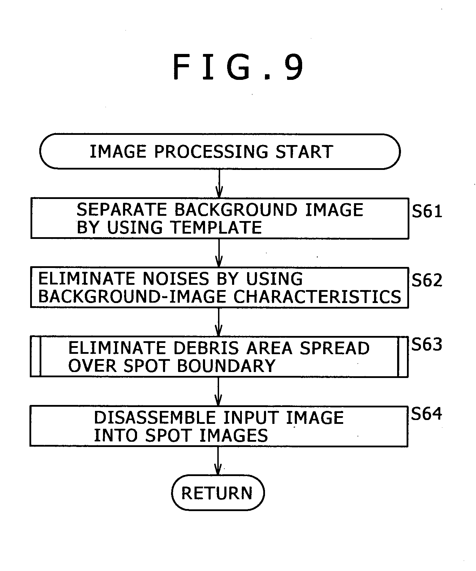

Fig. 9 shows a flowchart to be referred to in explanation of image processing.
[Fig. 10]

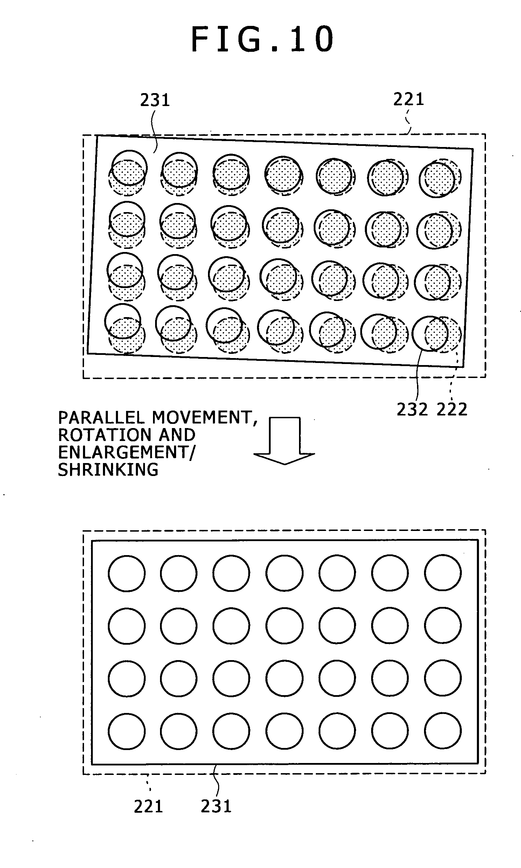

Fig. 10 is an explanatory diagram to be referred to in description of a process to extract an expression profile image by making use of a template.
[Fig. 11]

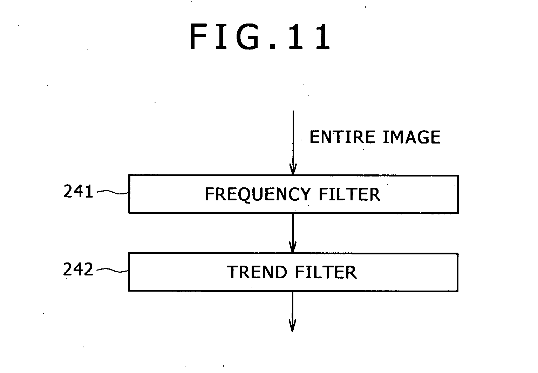

Fig. 11 is an explanatory diagram to be referred to in description of processing of filters.
[Fig. 12]

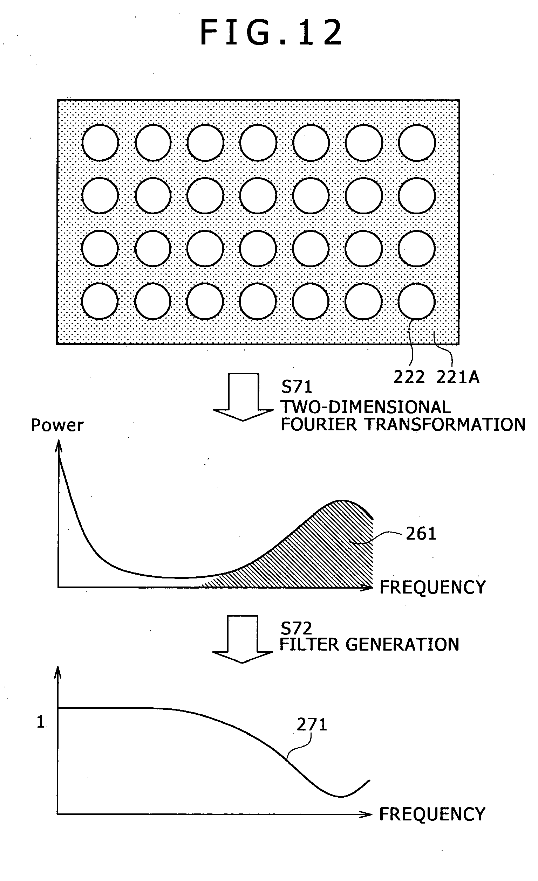

Fig. 12 is an explanatory diagram to be referred to in description of a process to generate a filter.
[Fig. 13]

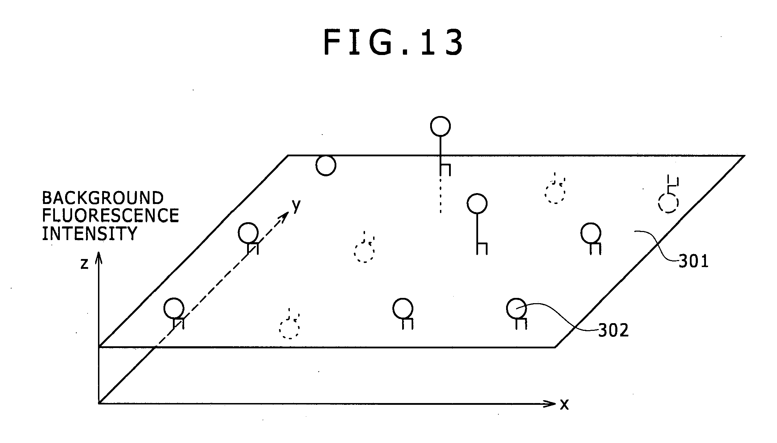

Fig. 13 is an explanatory diagram to be referred to in description of processing carried out by a trend filter.
[Fig. 14]

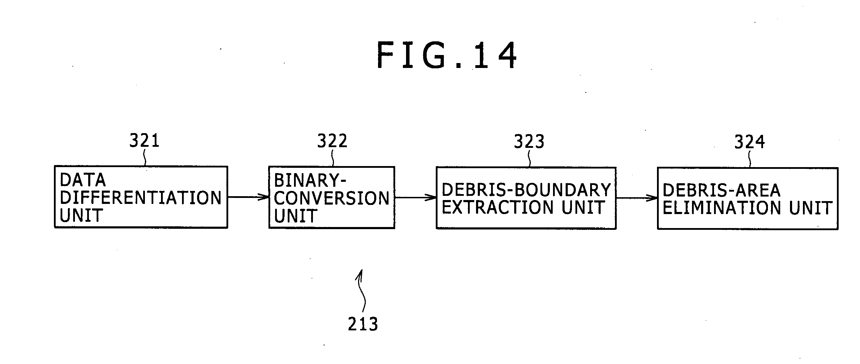

Fig. 14 is a block diagram showing a typical configuration of an area elimination unit.
[Fig. 15]

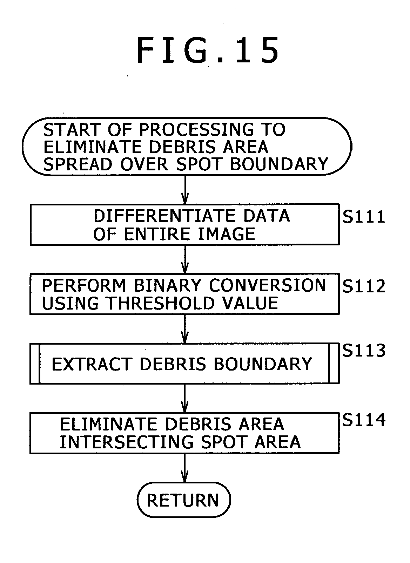

Fig. 15 shows a flowchart to be referred to in explanation of processing to eliminate a debris area spread over a spot boundary.
[Fig. 16]

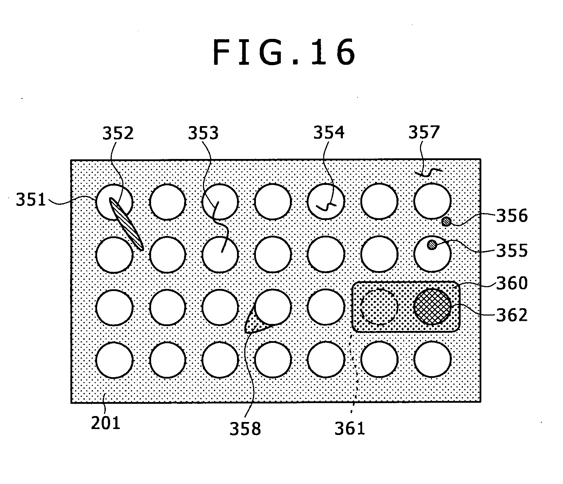

Fig. 16 is a diagram showing various typical kinds of debris.
[Fig. 17]
Fig. 17 is a diagram showing a binary-coded image. [Fig. 18]
Fig. 18 is a block diagram showing a typical configuration of a debris-boundary extraction unit.
[Fig. 19]

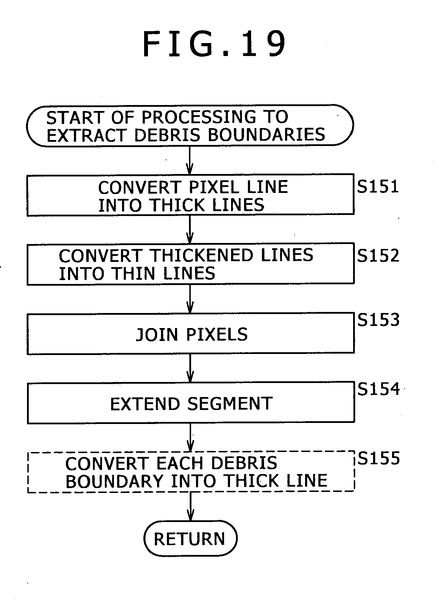

Fig. 19 shows a flowchart to be referred to in explanation of debris-boundary extraction processing.
[Fig. 20]

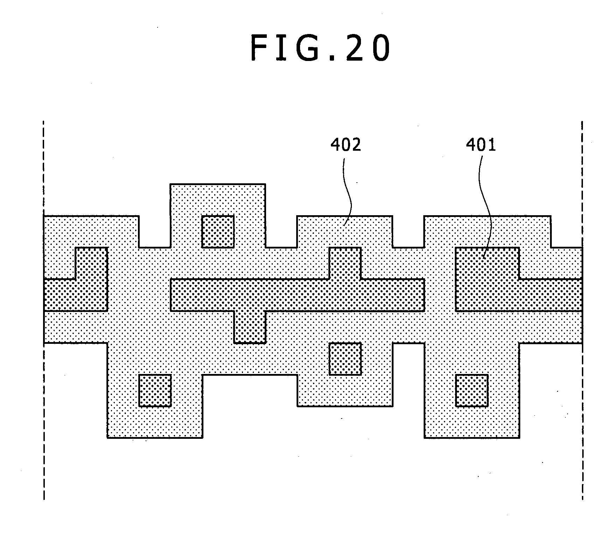

Fig. 20 is an explanatory diagram to be referred to in description of thick-line conversion processing.
[Fig. 21]

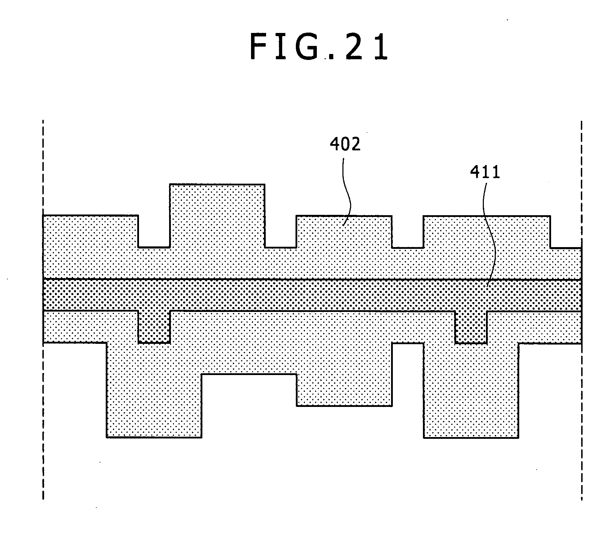

Fig. 21 is an explanatory diagram to be referred to in description of thin-line conversion processing.
[Fig. 22]

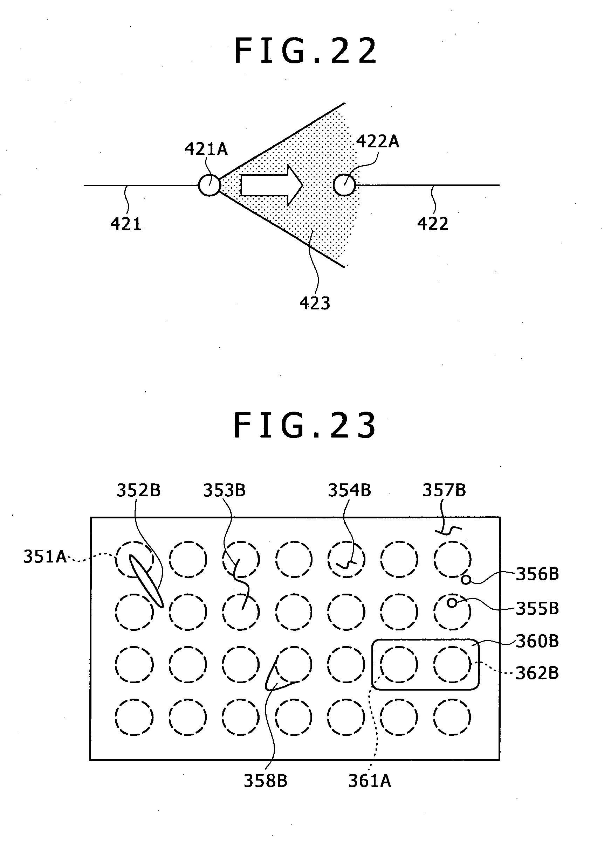

Fig. 22 is an explanatory diagram to be referred to in description of processing to extend a segment.
[Fig. 23]
Fig. 23 is an explanatory diagram to be referred to in description of a state in which each debris boundary has been made a thick line.
[Fig. 24]

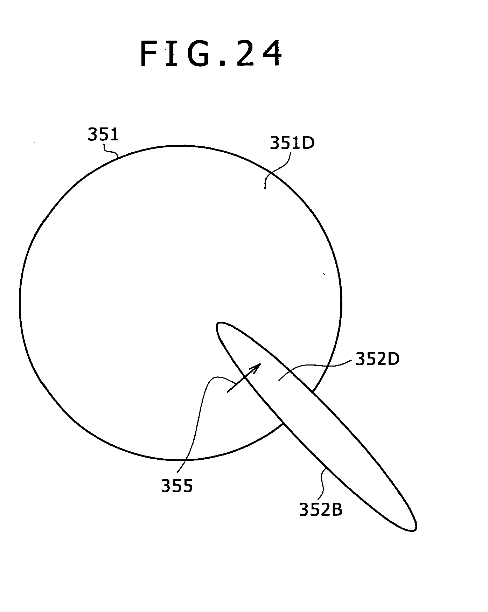

Fig. 24 is an explanatory diagram to be referred to in description of a debris area intersecting a spot boundary.
[Fig. 25]

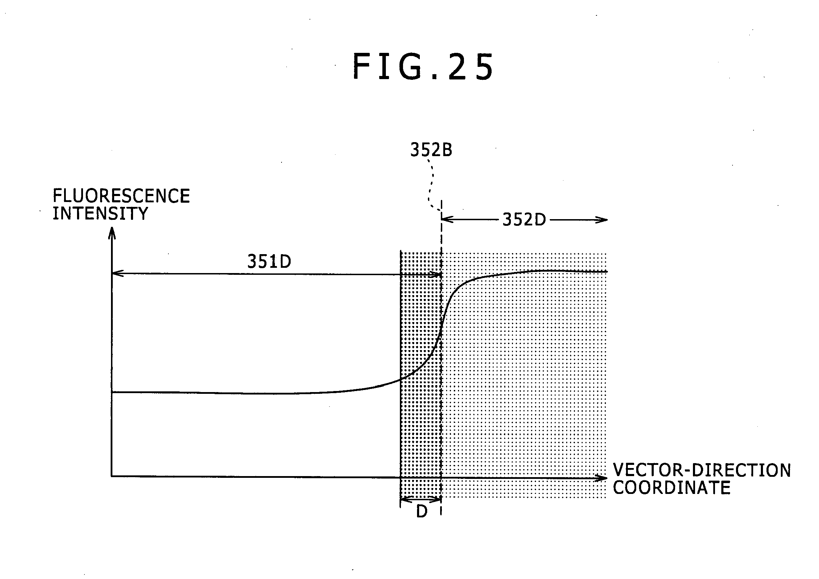

Fig. 25 is an explanatory diagram to be referred to in description of a boundary between a debris area and a spot internal area.
[Fig. 26]

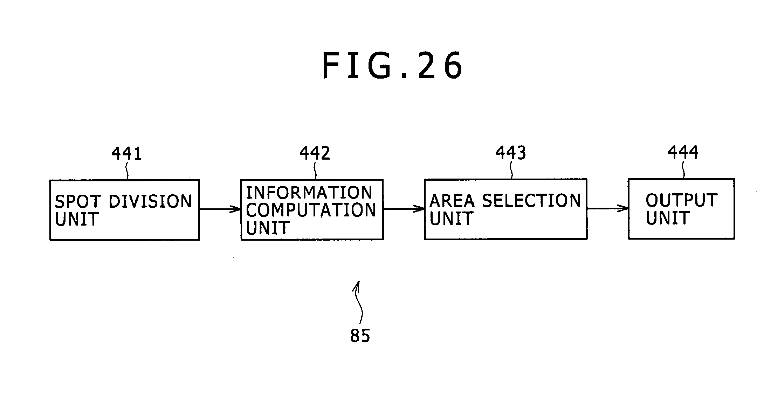

Fig. 26 is a block diagram showing a typical configuration of a hybridization-level computation unit.
[Fig. 27]

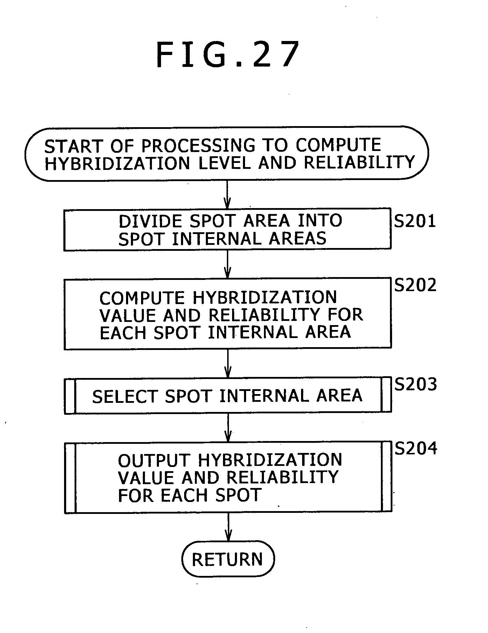

Fig. 27 shows a flowchart to be referred to in explanation of processing to compute a hybridization level and a reliability.
[Fig. 28]

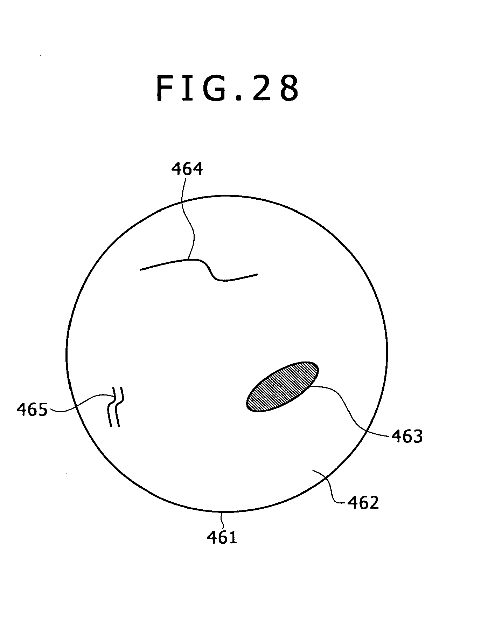

Fig. 28 is a diagram showing a typical image of a spot including debris.
[Fig. 29]

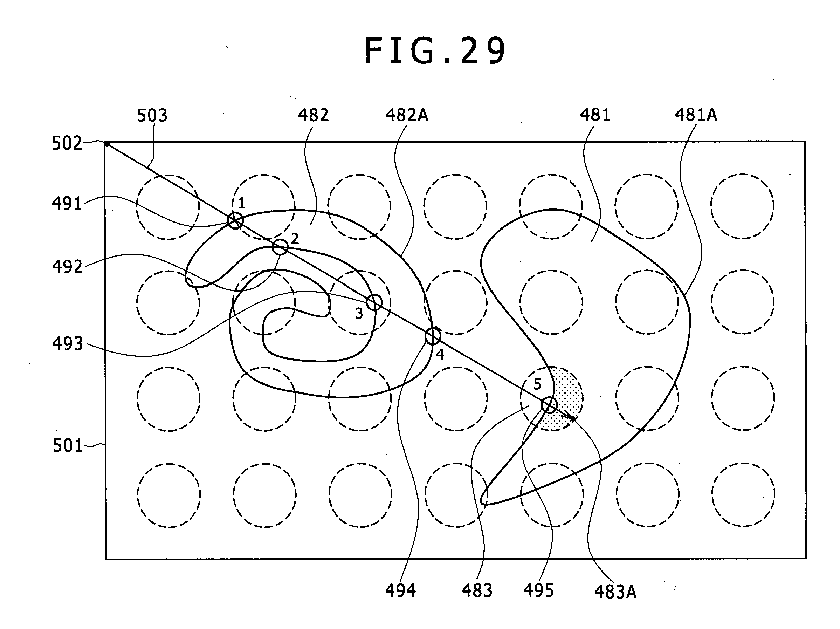

Fig. 29 is a diagram to be referred to in description of a principle to confirm that an observed point is inside of a debris area.
[Fig. 30]

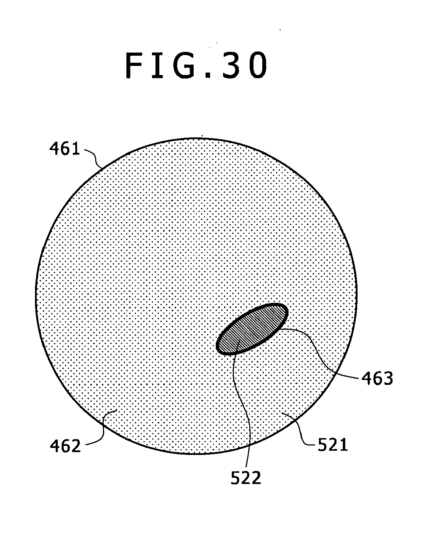

Fig. 30 is an explanatory diagram to be referred to in description of spot internal areas.
[Fig. 31]

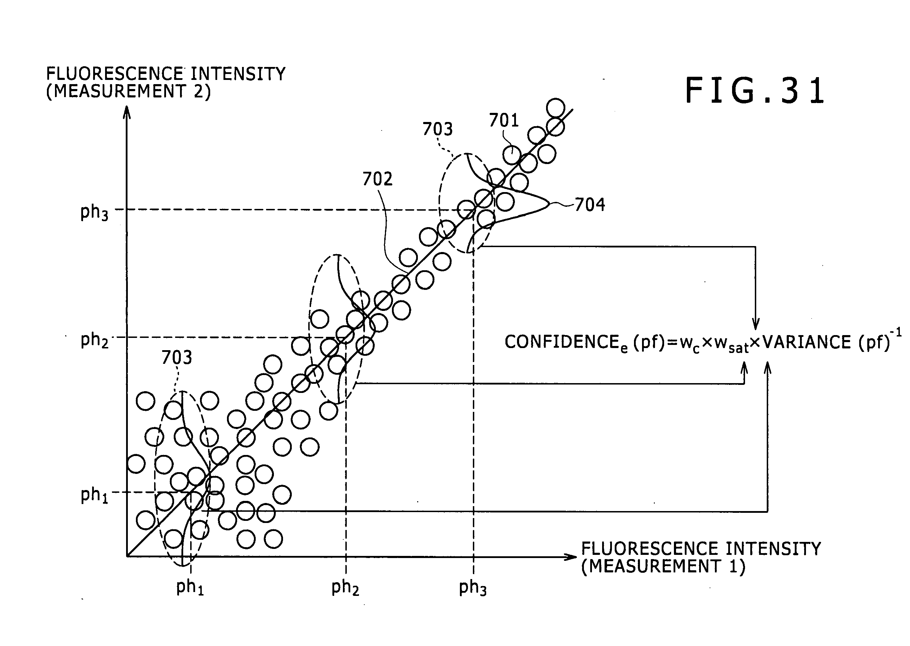

Fig. 31 is an explanatory diagram to be referred to in description of reliability confidence.
[Fig. 32]

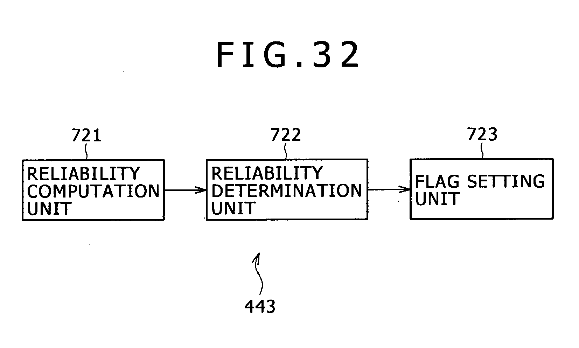

Fig. 32 is a block diagram showing a typical configuration of a selection unit.
[Fig. 33]

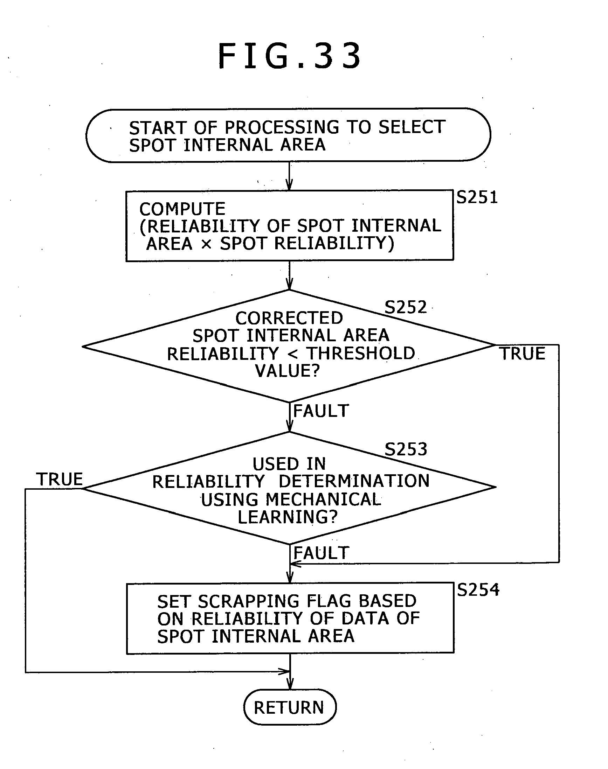

Fig. 33 shows a flowchart to be referred to in explanation of processing to select a spot internal area.
[Fig. 34]

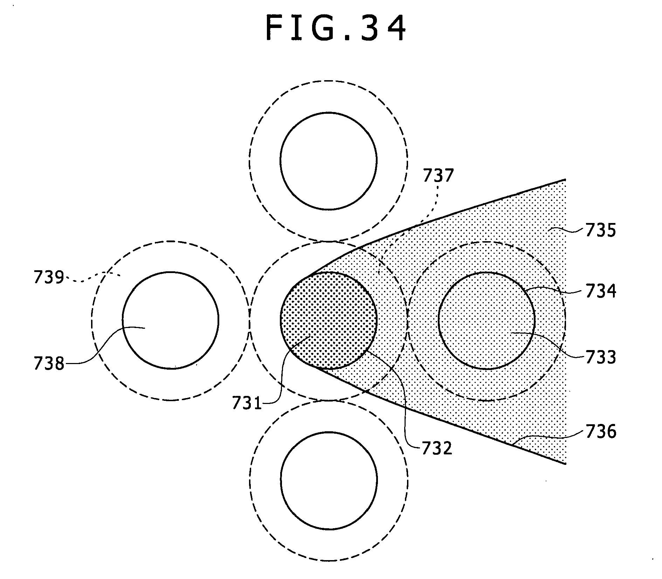

Fig. 34 is an explanatory diagram to be referred to in description of spot peripheries.
[Fig. 35]

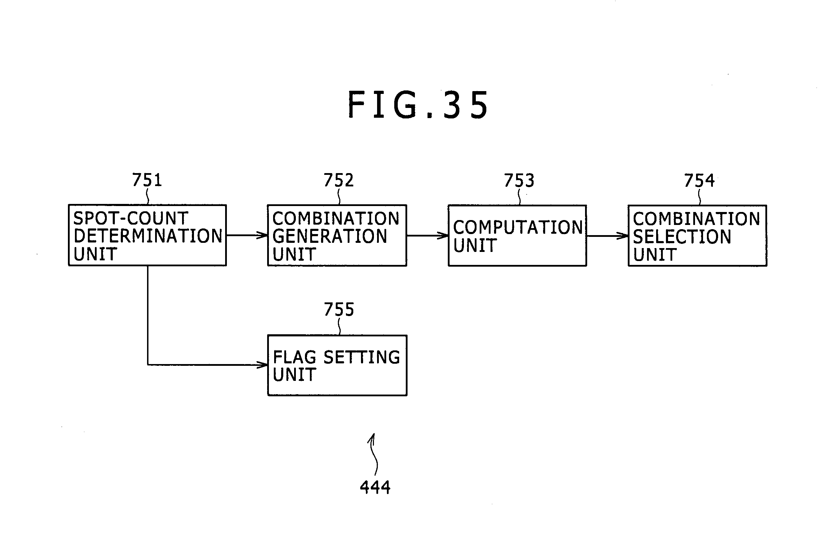

Fig. 35 is a block diagram showing a typical configuration of an output unit.
[Fig. 36]

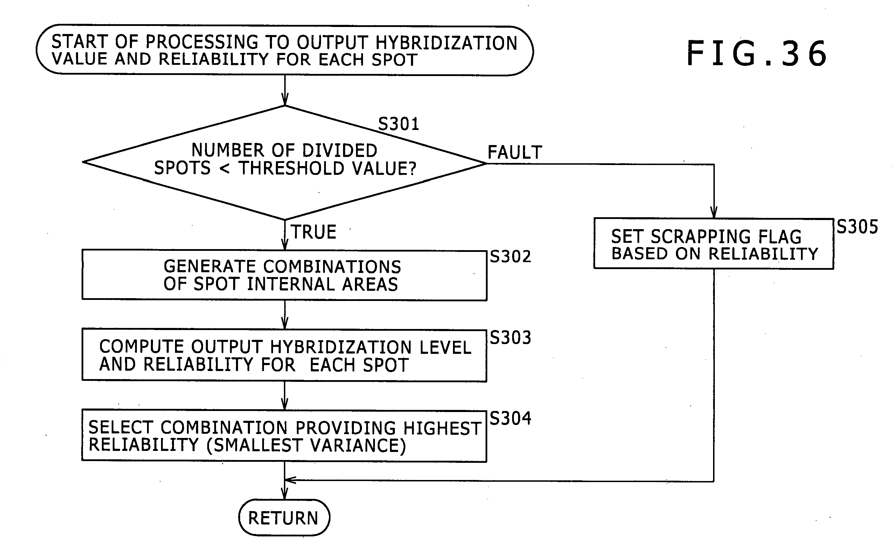

Fig. 36 shows a flowchart to be referred to in explanation of processing to output a hybridization value and a reliability for each spot.
[Fig. 37]

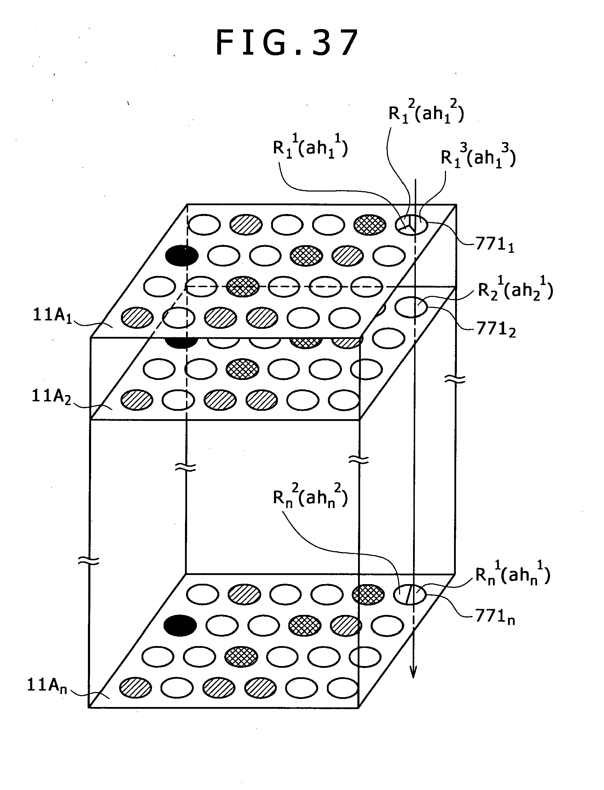

Fig. 37 is an explanatory diagram to be referred to in description of a configuration of spots obtained from a plurality of experiments.
[Fig. 38]

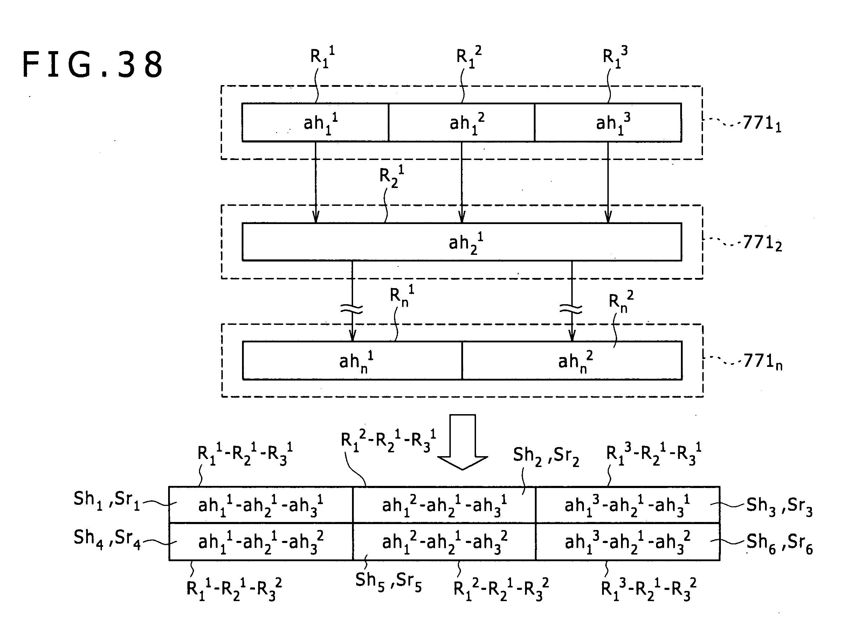

Fig. 38 is an explanatory diagram to be referred to in description of a typical combination of spot internal areas each picked up from one of a plurality of experiments.
[Fig. 39]

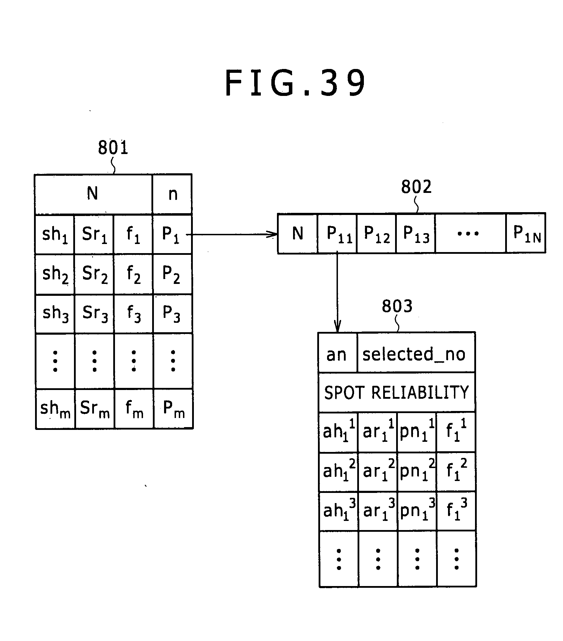

Fig. 39 is a diagram showing a typical configuration of expression profile data.
[Fig. 40]

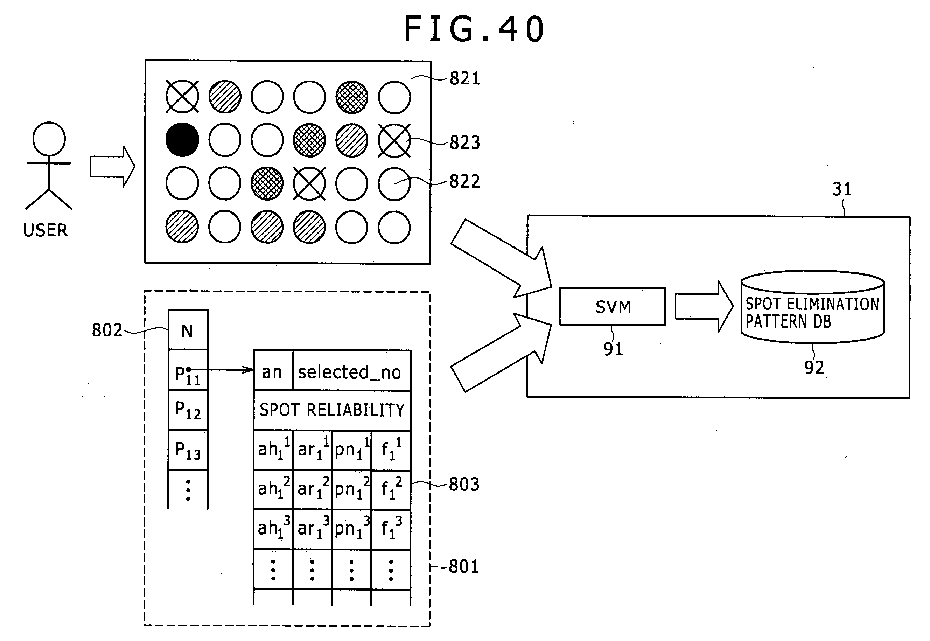

Fig. 40 is an explanatory diagram to be referred to in description of an SVM learning process.
[Fig. 41]

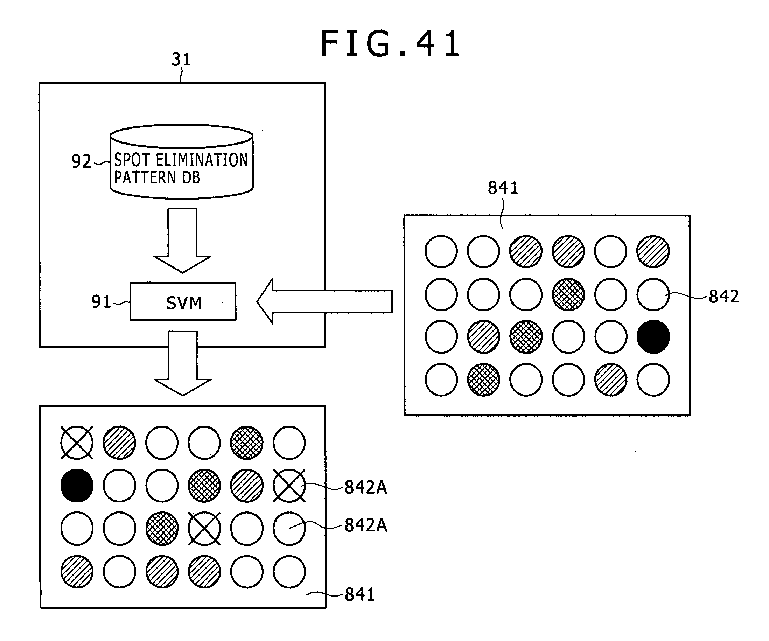

Fig. 41 is an explanatory diagram to be referred to in description of an SVM determination process.
[Fig. 42]

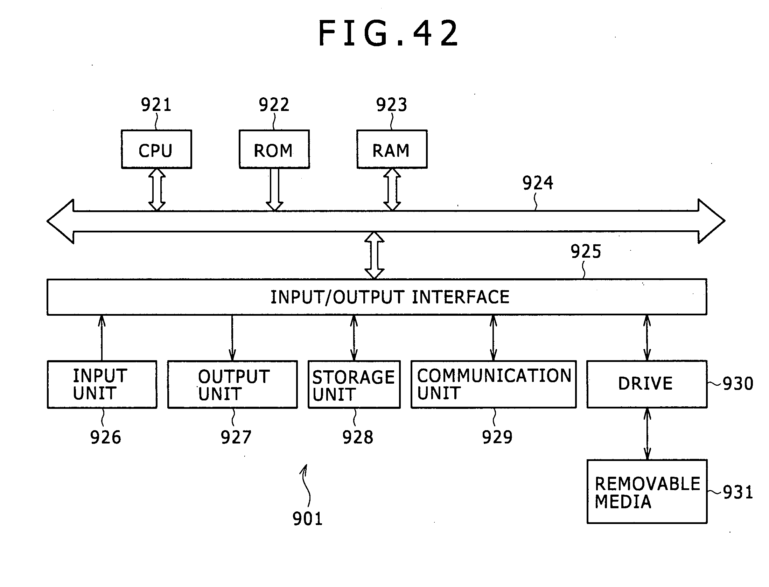

Fig. 42 is a block diagram showing a typical configuration of a personal computer.

[Description of Reference Symbols]

1: Biological-information processing apparatus
11: DNA chip
21: Pickup unit
22: Fluorescence-intensity acquisition unit
23: Excitation light intensity computation unit
24: Hybridization-level estimation unit
25: Expression-amount computation unit
28: Expression profile data storage unit
29: User-interface unit
30: Fluorescence-intensity/hybridization-level
conversion-equation storage unit
81: Excitation light intensity estimation unit
82: Creation unit
83: Image processing unit
84: Validation unit
85: Hybridization-level computation unit

[Best Modes for Carrying out the Invention]

The meanings of technical terms used in this description are explained as follows:
The term "probe" means a biological substance, which is immobilized on a substrate used as a base in creation of a bioassay such as a DNA chip and undergoes a bioreaction in conjunction with a target.
The term "target" means a biological substance, which undergoes a bioreaction in conjunction with another biological substance immobilized on a substrate used as a base in creation of a bioassay such as a DNA chip.
The term "biological substance" embraces genes having mutually complementary base sequences or substances derived from the genes in addition to substances formed in vivo such as proteins, nucleic acids and saccharides.
The term "bioreaction" means a chemical reaction which two or more biological substances biologically undergo. Its typical example is the hybridization.
The term "hybridization" means a complementary-chain (double-strand) forming reaction undergone by nucleic acids having complementary base sequence structures.
Fig. 1 is a block diagram showing a typical configuration of a biological-information processing apparatus according to an embodiment of the present invention. As shown in the figure, the biological-information processing apparatus 1 includes a DNA chip 11, a pickup unit 21, a fluorescence-intensity acquisition unit 22, an excitation light intensity computation unit 23, a hybridization-level estimation unit 24, an expression-amount computation unit 25, a data standardization unit 26, an output unit 27, an expression profile data storage unit 28, a user interface (UI) 29 having a display unit 29A, a fluorescence-intensity/hybridization-level conversion-equation storage unit 30 and a mechanical learning unit 31.

The DNA chip 11 has a spot 12 and a guide 13. Fig. 2 is a diagram showing a typical detailed configuration of the DNA chip 11.

As shown in the figure, the DNA chip 11 has an expression analysis reaction channel 101 and a cell-count computation reaction channel 102, which are laid on a substrate 11A. On the edge of the lower side shown in the figure as the lower side of the substrate 11A, a guide 13A having a straight-line shape is provided. On the edge of the upper side shown in the figure as the upper side of the substrate 11A, on the other hand, an end-position guide 13B is provided. To put it concretely, the start-position guide 13A and the end-position guide 13B jointly form the guide 13 shown in Fig. 1.

The expression analysis reaction channel 101 and the cell-count computation reaction channel 102 are located between the start-position guide 13A and the end-position guide 13B.

On the expression analysis reaction channel 101, a plurality of spots 12 are each formed as a reaction area. A hybridization validation probe 111, an expression analysis probe 112 and an expression standardization control probe 113 are immobilized on the expression analysis reaction channel 101. The hybridization validation probe 111 serves as a biological substance (a first biological substance). If a sample is dropped to the expression analysis reaction channel 101, a target 111A undergoes a hybridization in conjunction with the hybridization validation probe 111. The target 111A also serves as a biological substance (a second biological substance). The target 111A is a substance having a base forming a complementary structure in conjunction with the base of the hybridization validation probe 111. By the same token, a target 112A and the expression analysis probe 112 undergo a hybridization. Serving as a biological substance (a second biological substance), the target 112A is a substance having a base forming a complementary structure in conjunction with the base of the expression analysis probe 112. In the same way, a target 113A and the expression standardization control probe 113 undergo a hybridization. Serving as a biological substance (a second biological substance), the target 113A is a substance having a base forming a complementary structure in conjunction with the base of the expression standardization control probe 113.

On the cell-count computation reaction channel 102, a hybridization validation probe 114 and a cell-count computation control probe 115, which each serve as a biological substance (a first biological substance), are each attached to a spot 12. If a sample is dropped to the cell-count computation reaction channel 102, the hybridization validation probe 114 and a target 114A undergo a hybridization. Serving as a biological substance (a second biological substance), the target 114A is a substance having a base forming a complementary structure in conjunction with the base of the hybridization validation probe 114. By the same token, the cell-count computation control probe 115 and a target 115A undergo a hybridization. Serving as a biological substance (a second biological substance), the target 115A is a substance having a base forming a complementary structure in conjunction with the base of the cell-count computation control probe 115.

An intercalater 116 is joined to a probe and a target, which are used as biological substances completing a hybridization (or a bioreaction). The intercalater 116 generates fluorescent light when excitation light is radiated to the intercalater 116.

Fig. 2 is a diagram showing a state in which a target has undergone a hybridization in conjunction with a probe. For the sake of convenience, in Fig. 2, only one probe is shown for each spot. In actuality, however, a plurality of probes of the same type can be immobilized on one spot. In addition, any arbitrary number of spots are laid out at positions determined in advance on each reaction channel and, on each of the spots, a plurality of probes of the same type are immobilized.

As shown in Fig. 1, the pickup unit 21 includes a fluorescence-intensity acquisition pickup 41, a guide-signal acquisition pickup 42, a control unit 43, an object-coordinate computation unit 44 and a convolution development unit 45.

The fluorescence-intensity acquisition pickup 41 is a pickup for acquiring images of the expression analysis reaction channel 101 and the cell-count computation reaction channel 102 of the DNA chip 11. On the other hand, the guide-signal acquisition pickup 42 is a pickup for reading out a start-position guide 13A and an end-position guide 13B.

The fluorescence-intensity acquisition pickup 41 has an objective lens 51, a prism 52, a semiconductor laser 53 and a photodiode 54. A laser light beam (or an excitation light beam) generated by the semiconductor laser 53 propagates to the objective lens 51 by way of the prism 52. The objective lens 51 radiates the arriving laser light beam to the substrate 11A (or the spot 12). In addition, the objective lens 51 radiates a light beam coming from the spot 12 to the photodiode 54 by way of the prism 52. As described above, on each of the spots 12, a plurality of probes are immobilized and, when a probe and a target undergo a hybridization, an intercalater 116 is joined to both the probe and the target. That is to say, if a probe and a target do not undergo a hybridization, no intercalater 116 exists between the probe and the target. In other words, only if a probe and a target undergo a hybridization, an intercalater 116 does exist between both the probe and the target. As described earlier, the intercalater 116 generates fluorescent light when excitation light is radiated to the intercalater 116. A fluorescent light beam converged by the objective lens 51 is split by the prism 52 into excitation light beams which are then injected to the photodiode 54.

If the quantities of biological substances undergoing a hybridization are large, the quantity of a resulting intercalater 116 is also large, being proportional to the quantities of biological substances. Thus, on the basis of the fluorescence intensity of fluorescent light generated by the intercalater 116, the state of a hybridization can be measured (that is, information on the hybridization can be obtained).

The control unit 43 is a unit for controlling the current flowing through the semiconductor laser 53 so as to adjust the intensity of the excitation light generated by the semiconductor laser 53. The control unit 43 also retrieves the output (changes in current magnitude) of the photodiode 54.

The convolution development unit 45 receives a signal from the control unit 43 as a signal representing the current magnitude changes output by the photodiode 54 and generates pixel data for each pixel.

The guide-signal acquisition pickup 42 has an objective lens 61, a prism 62, a semiconductor laser 63 and a photodiode 64. The semiconductor laser 63 is a component for generating a laser light beam on the basis of control executed by the control unit 43. (This laser light beam functions as a guide detection light beam). The prism 62 is a component for injecting the laser light beam generated by the semiconductor laser 63 to the objective lens 61. The objective lens 61 is a lens for radiating the laser light beam to the substrate 11A. The objective lens 61 receives a light beam reflected by the substrate 11A, and the prism 62 outputs on the reflected light beam to the photodiode 64. The photodiode 64 is a component for optically converting the reflected light beam injected by the prism 62 into a guide signal and outputting the guide signal to the control unit 43. The control unit 43 passes on the guide signal received from the photodiode 64 to the object-coordinate computation unit 44. Guides 13 (that is, a start position guide 13A and an end position guide 13B) are each created to provide a high reflection rate (or a low reflection rate) in comparison with other areas on the substrate 11A. On the basis of the level of the guide signal received from the guide-signal acquisition pickup 42 through the control unit 43, the object-coordinate computation unit 44 computes the positions of the start-position guide 13A and the end-position guide 13B and also computes the position (or the coordinates) of the guide-signal acquisition pickup 42, which moves at a uniform velocity in the direction from the start-position guide 13A to the end-position guide 13B.

On the basis of a position computed by the object-coordinate computation unit 44 as the position of the guide-signal acquisition pickup 42, the control unit 43 controls the position of fluorescence-intensity acquisition pickup 41 (that is, the position of the objective lens 51). The guide-signal acquisition pickup 42 and the fluorescence-intensity acquisition pickup 41 are located at positions according to a constant positional relation determined in advance. Thus, placing fluorescence-intensity acquisition pickup 41 at a position between the start-position guide 13A and the end-position guide 13B shown in Fig. 2 causes the guide-signal acquisition pickup 42 to be placed at a predetermined position between the start-position guide 13A and the end-position guide 13B.

The fluorescence-intensity acquisition unit 22 is a section for receiving a fluorescence intensity ($pf_{x, y}$) output by the photodiode 54 employed in the fluorescence-intensity acquisition pickup 41 for each spot (or the coordinates (x, y) of each spot) and outputting data related to the fluorescence intensity to an excitation light intensity estimation unit 81 employed in the hybridization-level estimation unit 24. The fluorescence-intensity acquisition unit 22 also outputs control signals to the control unit 43 as signals to be used for controlling the object coordinates (x, y) and object area radius (r) of the objective lens 51 employed in fluorescence-intensity acquisition pickup 41 on the substrate 11A and for controlling the intensity of the excitation light beam. On the basis of these control signals, the control unit 43 controls the objective lens 51. To put it in detail, the objective lens 51 is placed at a position on the substrate 11A and radiates the excitation light beam to a radiation range on the substrate 11A at an intensity (or the intensity of the excitation light beam) adjusted to a value determined in advance. The position on the substrate 11A has the aforementioned object coordinates (x, y) determined in advance. The radiation range on the substrate 11A has the aforementioned object area radius r, which is controlled to a value determined in advance.

The fluorescence-intensity acquisition unit 22 passes on the fluorescence intensity received from the control unit 43 to the excitation light intensity computation unit 23. The excitation light intensity computation unit 23 is a section for computing an optimum excitation-light intensity and outputting the optimum excitation-light intensity obtained as a result of the computation to the fluorescence-intensity acquisition unit 22. The excitation light intensity computation unit 23 computes the optimum excitation-light intensity on the basis of the fluorescence intensity received from the fluorescence-intensity acquisition unit 22 at a pre-scan time in accordance with a conversion equation stored in a fluorescence-intensity/hybridization-level conversion-equation storage unit 30. At an actual scan time, the fluorescence-intensity acquisition unit 22 controls a current flowing to the semiconductor laser 53 on the basis of the optimum excitation-light intensity received from the excitation light intensity computation unit 23 in order to drive the semiconductor laser 53 to output an excitation light beam having an intensity determined in advance.

The hybridization-level estimation unit 24 includes the aforementioned excitation light intensity estimation unit 81, a creation unit 82, an image processing unit 83, a validation unit 84 and a hybridization-level computation unit 85.

The excitation light intensity estimation unit 81 is input means for inputting image information of a reaction area and carrying out processing to estimate the intensity of the excitation light if necessary. The image information includes image data based on the fluorescence intensity received from the fluorescence-intensity acquisition unit 22 or expression profile data stored in the expression profile data storage unit 28 in advance. The creation unit 82 is a section for creating an equation of the hybridization (pf) as an equation for unambiguously determining a hybridization level from the fluorescence intensity on the basis of data received from the excitation light intensity estimation unit 81. The image processing unit 83 is a section for processing image data received from the creation unit 82 and outputting the result of the processing to the validation unit 84 and the UI unit 29. The UI unit 29 displays an image based on the data received from the image processing unit 83 on the display unit 29A of the UI unit 29. In addition, in processing to be described later by referring to a flowchart shown in Fig. 9, the image processing unit 83 also carries out a process to eliminate debris portions from the image of a DNA chip 11 and a process to disassemble the image into images of spots 12 on the basis of an input entered by the user via the UI unit 29. The debris is a substance serving as an obstruction to a measurement.

The validation unit 84 is a section for confirming that a hybridization has been carried out correctly on the basis of hybridization levels included in image data received from the image processing unit 83 as hybridization quantities for spots 12 on hybridization validation probes 111 and 114.

The hybridization-level computation unit 85 is a section for dividing a spot area into spot internal areas, computing a hybridization value as well as a reliability for each of the spot internal areas and outputting the hybridization value as well as the reliability for each spot in processing to be explained later by referring to a flowchart shown in Fig. 27.

The expression amount computation unit 25 is a section for finding the strength of a junction between a probe and a target on the basis of the output of the hybridization-level computation unit 85 in order to estimate an expression amount for a fluorescence intensity. The data standardization unit 26 is a section for carrying out data standardization processing making use of the expression standardization control probe 113 and the cell-count computation control probe 115. The output unit 27 is a section for supplying standardized data to the expression profile data storage unit 28. The expression profile data storage unit 28 is a section for storing data received from the output unit 27 as expression profile data. If necessary, the data stored in the expression profile data storage unit 28 is supplied to be displayed on the display unit 29A. By the same token, if necessary, data output by the expression amount computation unit 25 is supplied to the UI unit 29 to be displayed on the display unit 29A.

The fluorescence-intensity/hybridization-level conversion-equation storage unit 30 is a section for storing a conversion equation unambiguously determining a relation between the fluorescence intensity and the hybridization level for the fluorescence intensity in advance as will be described later by referring to Fig. 7. The relation is not necessarily expressed by a conversion equation. That is to say, data expressing the relation may also be stored in the fluorescence-intensity/hybridization-level conversion-equation storage unit 30 in advance.

The mechanical learning unit 31 has an SVM (Support Vector Machine) 91 serving as mechanical learning means and a spot elimination pattern DB 92. The SVM 91 is a unit for carrying out a learning process in a learning mode on the basis of data received from the UI unit 29 and the expression profile data storage unit 28, and storing the result of the learning process in the spot elimination pattern DB 92. In a determination mode, on the other hand, the SVM 91 determines data received from the expression profile data storage unit 28 on the basis of a pattern stored in the spot elimination pattern DB 92 and supplies the result of the determination to the hybridization-level computation unit 85.

A process to quantitatively measure a gene expression amount is carried out by an experimental-process processing apparatus 131 shown in Fig. 3. The biological-information processing apparatus 1 shown in Fig. 1 is an apparatus forming a portion of the experimental-process processing apparatus 131 shown in Fig. 3.

As shown in Fig. 3, the experimental-process processing apparatus 131 employs a target preparation unit 141, a hybridization unit 142, a fluorescence-intensity acquisition unit 143, an expression-amount estimation unit 144, a data standardization unit 145, an output unit 146 and a storage unit 147. The fluorescence-intensity acquisition unit 143, the expression-amount estimation unit 144, the data standardization unit 145, the output unit 146 and the storage unit 147 carry out the functions of the biological-information processing apparatus 1 shown in Fig. 1. To put it concretely, the fluorescence-intensity acquisition unit 143 carries out the functions of the pickup unit 21, the fluorescence-intensity acquisition unit 22, the excitation light intensity computation unit 23 and the fluorescence-intensity/hybridization-level conversion-equation storage unit 30. The expression amount estimation unit 144 carries out the functions of the hybridization-level estimation unit 24, the expression amount computation unit 25 and the mechanical learning unit 31. The data standardization unit 145 carries out the functions of the data standardization unit 26. The output unit 146 carries out the functions of the output unit 27 and the storage unit 147 carries out the functions of the expression profile data storage unit 28.

The target preparation unit 141 is a section for preparing a target. The hybridization unit 142 is a section for accomplishing a hybridization of a probe and a target. The fluorescence-intensity acquisition unit 143 is a section for acquiring a fluorescence intensity. The expression amount estimation unit 144 is a section for carrying out a process to estimate an expression amount. The data standardization unit 145 is a section for standardizing data. The output unit 146 is a section for outputting expression profile data. The storage unit 147 is a section for storing the expression profile data.

Next, the processing carried out by the experimental-process processing apparatus 131 shown in Fig. 3 is explained by referring to a flowchart shown in Fig. 4 as follows.

As shown in the figure, the flowchart begins with a S11 at which the target preparation unit 141 prepares a target. To put it concretely, samples each including a cell are retrieved and processing to alter proteins in order to eliminate the proteins from the samples is carried out. Then, an RNA (ribonucleic acid) is extracted and fractionated into fragments and, by the same token, a DNA (deoxyribonucleic acid) is also extracted and fractionated into fragments in order to generate a target, which is the aforementioned target 112A for the expression analysis probe 112.

Then, at the next step S12, the hybridization unit 142 carries out a hybridization. To put it concretely, targets 111A and 114A for hybridization validation probes 111 and 114 respectively, a target 113A for an expression standardization control probe 113 and a target 115A for a cell-count computation probe 115 are added to a liquid containing the targets generated in the process carried out at the step S11. Then, the liquid is dropped on the expression analysis reaction channel 101 and the cell-count computation reaction channel 102 in order to hybridize the targets with the probes. Then, intercalaters 116 are introduced to the targets hybridized with the probes, being joined to the targets and the probes, in order to generate a DNA chip 11 like the one shown in Fig. 2. As shown in the same figure, the expression analysis probe 112 has been hybridized with the target 112A on a spot 12 of the expression analysis reaction channel 101. In addition, the expression standardization control probe 113 has been hybridized with the target 113A on a spot of the expression analysis reaction channel 101 whereas the hybridization validation probe 111 has been hybridized with the target 111A on a spot of the expression analysis reaction channel 101. An intercalater 116 has been joined to every probe, which has undergone a double-strand joining process in conjunction with a target.

By the same token, the hybridization validation probe 114 has been hybridized with the target 114A on a spot 12 of the cell-count computation reaction channel 102 whereas the cell-count computation control probe 115 has been hybridized with the target 115A on a spot of the cell-count computation reaction channel 102. In addition, in the same way, an intercalater 116 has been joined to every probe, which has undergone a double-strand joining process in conjunction with a target.

Then, at the next step S13, the fluorescence-intensity acquisition unit 143 acquires a fluorescence intensity. To put it concretely, the fluorescence-intensity acquisition unit 22 drives fluorescence-intensity acquisition pickup 41 through the control unit 43 to have the semiconductor laser 53 generate a laser light beam as an excitation light beam. The excitation light beam is injected to the objective lens 51 by way of the prism 52, and the objective lens 51 then radiates the excitation light beam to the expression analysis reaction channel 101 on the substrate 11A.

As described before, an intercalater 116 generates fluorescent light when excitation light is radiated to the intercalater 116. The objective lens 51 converges the fluorescent light generated by the intercalater 116 and injects a converged fluorescent light beam to the photodiode 54 by way of the prism 52. Receiving the fluorescent light beam, the photodiode 54 outputs a current corresponding to the fluorescent light beam to the control unit 43. The control unit 43 outputs a signal corresponding to the current to the convolution development unit 45 for converting the signal into an image signal. The convolution development unit 45 outputs the image signal resulting from the conversion process as a signal corresponding to the fluorescence intensity to the fluorescence-intensity acquisition unit 22 by way of the control unit 43.

The control unit 43 moves the position of the objective lens 51 in the direction from the start-position guide 13A to the end-position guide 13B. At that time, a laser light beam output by the semiconductor laser 63 employed in the guide-signal acquisition pickup 42 as a guide detection light beam is injected to the objective lens 61 by way of the prism 62 and the objective lens 61 radiates the guide detection light beam to the substrate 11A. The intensity of a reflected light beam obtained as a result of reflection of the guide detection light beam increases when the guide detection light beam hits the start-position guide 13A and the end-position guide 13B. The reflected light beam is injected to the prism 62 by way of the objective lens 61 whereas the prism 62 injects the reflected light beam to the photodiode 64. The photodiode 64 outputs a guide signal to the object-coordinate computation unit 44 by way of the control unit 43. On the basis of this guide signal, the object-coordinate computation unit 44 identifies the position of the guide-signal acquisition pickup 42 between the start-position guide 13A and the end-position guide 13B, which are provided on the substrate 11A, and computes the coordinates of the position. (It is to be noted that, since fluorescence-intensity acquisition pickup 41 is physically integrated with the guide-signal acquisition pickup 42 to form an assembly, the position of the guide-signal acquisition pickup 42 can also be regarded as the position of fluorescence-intensity acquisition pickup 41). The control unit 43 moves the guide-signal acquisition pickup 42 (along with fluorescence-intensity acquisition pickup 41) from the start-position guide 13A to the end-position guide 13B at a constant velocity (in a scanning operation).

After fluorescence-intensity acquisition pickup 41 is moved from the start-position guide 13A to the end-position guide 13B in a scanning operation shown in Fig. 2 as described above, the scan position is moved in the x-axis direction parallel to the start-position guide 13A (and the end-position guide 13B) by a distance equivalent to the size of a pitch. At this new scan position, the fluorescence-intensity acquisition pickup 41 is again moved from the start-position guide 13A to the end-position guide 13B. In this way, the expression analysis reaction channel 101 and the cell-count computation reaction channel 102 are entirely scanned and fluorescence-intensity acquisition pickup 41 outputs an image signal for every coordinates.

Then, at the next step S14, the expression amount estimation unit 144 carries out a process to estimate an expression amount. As will be described later in detail by referring to a flowchart shown in Fig. 5, in the process to estimate an expression amount, a hybridization level and a reliability are computed.

Then, at the next step S15, the data standardization unit 145 (or, to be more specific, the data standardization unit 26) carries out processing to standardize data. The processing to standardize data includes a standardization process based on the expression standardization control probe 113 and a standardization process based on the cell-count computation control probe 115. The standardization process based on the expression standardization control probe 113 is carried out as follows. Fig. 2 shows only one expression standardization control probe 113 at a position. In actuality, however, more than one expression standardization control probe 113 are located at a plurality of distributed positions. (For example, expression standardization control probes 113 are located respectively at five positions, i. e., positions at four corners of the expression analysis reaction channel 101 and the center of the expression analysis reaction channel 101). First of all, a correction curved surface such as a B spline curved surface is found on the basis of the fluorescence value of an expression standardization control probe 113 at each of the positions. Then, on the basis of the curved surface, a fluorescence value is found. Finally, the fluorescence value of each pixel is standardized by dividing the fluorescence value of the pixel by the fluorescence value found on the basis of the correction curved surface. With this standardization process, hybridization variations at different locations of spots 12 in the expression analysis reaction channel 101 can be corrected.

On the other hand, the standardization process based on the cell-count computation control probe 115 is carried out by dividing the fluorescence value of every pixel on each spot 12 in the cell-count computation reaction channel 102 by a fluorescence value based on the cell-count computation control probe 115. The fluorescence value based on the cell-count computation control probe 115 is the value of the hybridization level for the cell-count computation control probe 115. As the cell-count computation control probe 115, a reiteration sequence in a vivo genome extracting the expression analysis probe 112 is used. In the case of a human being, an example of such a reiteration sequence is the Alu sequence. With this standardization process, the expression amount of the acquired gene can be converted into a value per constant cell count.

Then, at the next step S16, the output unit 146 (or the output unit 27) outputs expression profile data. To put it concretely, the output unit 146 outputs the image data obtained as described above to the storage unit 147 (or the expression profile data storage unit 28).

Next, the processing carried out at the step S14 of the flowchart shown in Fig. 4 to estimate an expression amount is explained by referring to a flowchart shown in Fig. 5 as follows. The flowchart shown in Fig. 5 begins with a step S31 at which the excitation light intensity estimation unit 81 inputs image information. To put it concretely, the excitation light intensity estimation unit 81 inputs image information from the fluorescence-intensity acquisition unit 22. Then, at the next S32, the excitation light intensity estimation unit 81 produces a result of determination as to whether or not the image information input in the process carried out at the step S31 includes excitation light intensity information.

Fig. 6 is a diagram showing the format of the image data. The excitation light intensity estimation unit 81 inputs the data from the fluorescence-intensity acquisition unit 22. As shown in the figure, the image data input from the fluorescence-intensity acquisition unit 22 is composed of image data 181 and common data 182. The image data 181 includes the number of images forming a set. The image data 181 also includes the excitement light intensity, the number of horizontally and vertically arranged pixels and the fluorescent image for the image at each spot. The common data 182 includes a spot position template image, the number of spots and a probe gene index.

Thus, at least, in the case of image data supplied by the fluorescence-intensity acquisition unit 22, the excitement light intensity is included in the image data 181. Therefore, the determination result indicates that the image data includes excitation light intensity information. If an expression profile image is supplied from the expression profile data storage unit 28 used for storing such an image, on the other hand, the expression profile image may have been originated from the fluorescence-intensity acquisition unit 22 and stored in the expression profile data storage unit 28. Thus, if the expression profile image supplied from the expression profile data storage unit 28 is an image originated from the fluorescence-intensity acquisition unit 22, the image information includes excitation light intensity information. If the image information has been originated from another apparatus, on the other hand, the image information does not include excitation light intensity information in some cases.

If the determination result produced in the process carried out at the step S32 indicates that the image information input in the process carried out at the step S31 does not include excitation light intensity information, the flow of the processing represented in this flowchart goes on to a step S33 at which the excitation light intensity estimation unit 81 carries out a process to estimate an excitation light intensity.

The process to estimate an excitation light intensity can be carried out for image data measured on the basis of at least two excitation light beams having different light intensities. Thus, if image information measured on the basis of at least two excitation light beams having different light intensities does not exist, the process to estimate an excitation light intensity cannot be carried out. In this case, the process of the step S33 is skipped even if no excitation light intensity information is included in the received image data.

Then, at the next S34, the creation unit 82 produces a result of determination as to whether or not the input image information is information of an image taken in a photographing operation carried out by making use of a plurality of excitation light intensities. If the determination result produced in the process carried out at the step S34 indicates that the input image information is information of an image taken in a photographing operation by making use of a plurality of excitation light intensities, the flow of the processing represented by this flowchart goes on to a step S35. The creation unit 82 creates Eq. (1) shown below as an expression for determining a hybridization level (hybridization (pf)) on the basis of fluorescence intensities.

[Formula 1]

$$hybridization(pf) = (w_s + w_w)^{-1} \times \left\{ w_s \times hybridization_s(pf_s) + w_w \times hybridization_w(pf_w) \right\} \quad \ldots(1)$$

$$w_s = \begin{cases} 1 & : pf_s < lower_s \\ \dfrac{upper_s - pf_s}{upper_s - lower_s} & : lower_s \le pf_s < upper_s \\ 0 & : upper_s \le pf_s \end{cases} \quad \ldots(2)$$

$$W_w = \begin{cases} 0 & : pf_w < lower_w \\ \dfrac{pf_w - lower_w}{upper_w - lower_w} & : lower_w \le pf_w < upper_w \\ 1 & : upper_w \le pf_w \end{cases} \quad \ldots(3)$$

If the determination result produced in the process carried out at the step S34 indicates that the input image information is not information of an image taken in a photographing operation by using a plurality of excitation light intensities, on the other hand, the step S35 is skipped because the process of the step S35 cannot be carried out.

Fig. 7 is a diagram showing relations expressed by the expression $hybridization_e$ (pf) described above as relations between the fluorescence intensity and the hybridization level for each spot. As shown in the figure, for a given fluorescence intensity, the hybridization level is determined unambiguously on the basis of one of functions represented by curves 191 to 194. (That is to say, the hybridization information is determined unambiguously on the basis of a function of fluorescence intensity, which is obtained as a result of hybridization undergone by a first biological substance in conjunction with a second biological substance.) As shown in the figure, however, the curve 191 on the uppermost side of the figure is a curve for the smallest excitation light intensity. The curve 192 below the curve 191 is a curve for a relatively small excitation light intensity. The curve 193 below the curve 192 is a curve for a relatively large excitation light intensity. The curve 194 on the lowermost side of the figure is a curve for the largest excitation light intensity.

The expression $hybridization_s$ ($pf_s$) of Eq. (1) is the aforementioned expression $hybridization_e$ (pf) for a relatively large excitation light intensity among excitation light intensities used for generating data. On the other hand, the expression $hybridization_w$ ($pf_w$) of Eq. (1) is the aforementioned expression $hybridization_e$ (pf) for a relatively small excitation light intensity among excitation light intensities used for generating data.

Along segments 191A to 194A serving as an extension of the curves 191 to 194 on the left side of the figure and segments 191B to 194B serving as an extension of the same curve on the right side of the figure, for a small change in fluorescence intensity, the hybridization level varies very considerably. Thus, the segments 191A to 194A and 191B to 194B are excluded from the processing to compute a hybridization level from a fluorescence intensity. That is to say, only the middle portions of the curves 191 to 194 are used in the computation processing.

Then, at the next S36, the image processing unit 83 carries out image processing, details of which will be described later by referring to a flowchart shown in Fig. 9. In the image processing, a debris area spread over a spot boundary is eliminated from the image of the DNA chip 11 and the image is decomposed into image portions each corresponding to a spot.

Then, at the next step S37, the validation unit 84 carries out a process to validate the hybridization. As shown in Fig. 2, the hybridization validation probe 111 is immobilized on a spot 12 in the expression analysis reaction channel 101 whereas the hybridization validation probe 114 is immobilized on a spot 12 in the cell-count computation reaction channel 102. As each of the hybridization validation probe 111 and the hybridization validation probe 114, a gene sequence not of the type of the biological substance serving as an object of experiments is used. If the object of experiments is an animal, (that is, if the expression analysis probe 112 is the gene of an animal), for example, as each of the hybridization validation probe 111 and the hybridization validation probe 114, the gene of chlorophyll of a plant is used. As the targets 111A and 114A, a substance having a base forming a complementary structure in conjunction with the base of the hybridization validation probes 111 and 114 is used respectively. That is to say, a probe and a target, which are reliably capable of undergoing a hybridization without regard to the hybridization undergone by the expression analysis probe 112 in conjunction with the target 112A, are used as the hybridization validation probes 111 and 114 and the targets 111A and 114A respectively. Since the types of the used probes and the used targets are entirely different from the type of the objects of experiments, if the hybridization validation probe 111 and the hybridization validation probe 114 undergo a sufficient hybridization, the hybridization is proven to occur with a high degree of reliability in this experiment (or this measurement). On the other hand, the hybridization validation probe 111 and the hybridization validation probe 114 not undergoing a sufficient hybridization indicate that it is quite within the bounds of possibility that the measurement has been put in an environment making the hybridization difficult to occur for some reasons. Thus, the fluorescence value of each of the hybridization validation probe 111 and the hybridization validation probe 114 is measured and compared with a threshold value determined in advance. If the measured fluorescence value is at least equal to the predetermined threshold value, the hybridization is validated as a process that has occurred correctly.

Then, at the next step S38, the hybridization-level computation unit 85 computes a hybridization level and a reliability. As will be described in detail later by referring to a flowchart shown in Fig. 27, in the process to compute a hybridization level and a reliability, if debris exists in a spot area, the spot area is divided into a plurality of spot internal areas. Then, a hybridization level and a reliability are computed for each of the spot internal areas. Finally, a hybridization level and a reliability are computed for each spot.

Then, at the next step S39, the expression amount computation unit 25 carries out a process to compute an expression amount on the basis of the hybridization levels and the reliabilities. The hybridization levels and the reliabilities have been computed by the hybridization-level computation unit 85 in the process carried out at the step S38. In the process carried out at the step S39, an expression amount is computed for a calculated (acquired) fluorescence value.

Fig. 8 is a block diagram showing a typical functional configuration of the image processing unit 83. As shown in the figure, the functional configuration of the image processing unit 83 employs a background-image separation unit 211, a noise elimination unit 212, a debris-area elimination unit 213 and an image decomposition unit 214.

The background-image separation unit 211 is a section for separating a background image by making use of a template. The noise elimination unit 212 is a section for eliminating noises by making use of characteristics of the background image. The debris-area elimination unit 213 is a section for eliminating a debris area spread over a spot boundary. The image decomposition unit 214 is a section for decomposing an input image into spot images.

By referring to the flowchart shown in Fig. 9, the following description explains details of the image processing carried out by the image processing unit 83 at the step S36 of the flowchart shown in Fig. 5. The flowchart shown in Fig. 9 begins with a step S61 at which the background-image separation unit 211 separates a background image by making use of a template. Fig. 10 is a diagram showing the principle of the process to separate a background image. As shown in Fig. 10, a template 231 is prepared for an expression profile image 221 input from the equation creation unit 82. The template 231 has a plurality of spot positions 232. The spot positions 232 coincide respectively with a plurality of spot positions 222 in the expression profile image 221. That is to say, since the spot positions 222 of the expression profile image 221 are known, the template 231 having spot positions 232 each coinciding with one of the spot positions can be prepared.

As shown in a drawing on the lower side of Fig. 10, with respect to the expression profile image 221, the template 231 is subjected to a parallel shift, rotated, enlarged and/or contracted in order to make the spot positions 232 each coincide with one of the spot positions 222. With the spot positions 232 each coinciding with one of the spot positions 222, the images of the spot positions 232 are eliminated from the expression profile image 221 in order to separate the background image (that is, in order to extract only the background image).

Then, at the next step S62, the noise elimination unit 212 eliminates noises by making use of the characteristic of the background image. To put it concretely, the noise elimination unit 212 generates a frequency filter 241 and a trend filter 242 as shown in Fig. 11, applying the filters to the image data in order to eliminate the noises.

The frequency filter is generated as shown in Fig. 12. As shown in the figure, at a step S71, the noise elimination unit 212 applies the two-dimensional Fourier transformation to the data of a background image 221A having spot positions 222 in order to obtain frequency data. (The background image 221A is the background image extracted in the process carried out at the step S61). Then, at the next step S72, on the basis of the frequency data obtained as a result of the two-dimensional Fourier transformation, the noise elimination unit 212 generates a filter for eliminating high-frequency components 261. To be more specific, the spot 12 generates a filter having a characteristic represented by a characteristic curve 271 shown in Fig. 12 as a curve for eliminating the high-frequency components 261. Then, by applying the frequency filter 241 generated in this way to the image data, the noise elimination unit 212 is capable of eliminating noises caused by the high-frequency components from the image data.

Fig. 13 is a diagram showing a model of the principled configuration of the trend filter 242. As shown in the figure, the z axis of a three-dimensional coordinate space represents the fluorescence intensity of each pixel at a position prescribed by the (x, y) coordinates in the three-dimensional coordinate space. A regression plane 301 parallel to a horizontal (x, y) plane is used in the processing to eliminate noises on the basis of the fluorescence intensity of every pixel on the regression plane 301. By applying the trend filter 242 to the image data and making use a distance from the regression plane 301 as a quantity representing the fluorescence intensity 302 of a pixel at the position of the (x, y) coordinates, it is possible to eliminate noises caused by low-frequency components from the image data.

Then, at the next step S63, the debris-area elimination unit 213 carries out processing to eliminate a debris area spread over a spot boundary. The processing to eliminate a debris area spread over a spot boundary will be described later in detail by referring to a flowchart shown in Fig. 15. In this processing, a debris boundary is extracted and a debris area intersecting a spot boundary is eliminated.

Then, at the next step S64, the image decomposition unit 214 carries out a process to decompose an input image into spot images. To be more specific, the image decomposition unit 214 decomposes an image with its debris area eliminated in the process carried out at the step S63 into images of spots.

By referring to the flowchart shown in Fig. 15, the following description explains details of the processing carried out at the step S63 of the flowchart shown in Fig. 9 as processing to eliminate a debris area spread over a spot boundary. In order to carry out the processing, the debris-area elimination unit 213 in Fig. 8 is provided with functional sections such as a data differentiation unit 321 a binary-conversion unit 322, a debris-boundary extraction unit 323 and a debris-area elimination unit 324 as shown in Fig. 14.

The data differentiation unit 321 is a section for differentiating the data of the entire image. The binary-conversion unit 322 is a section for converting image data into binary values by making use of a threshold value. The debris-boundary extraction unit 323 is a section for extracting a debris boundary. The debris-area elimination unit 324 is a section for eliminating a debris area.

The flowchart shown in Fig. 15 begins with a step S111 at which the data differentiation unit 321 differentiates the data of the entire image. Then, at the next step S112, the binary-conversion unit 322 carries out processing to convert image data generated as a result of the differentiation process carried out at the step S111 into binary values by making use of a threshold value determined in advance.

Let us assume as an example a profile image 201 shown in Fig. 16. The profile image 201 has a spot boundary 351 for every spot. Various kinds of debris intersect spot boundaries 351 or exist inside spots. In the profile image 201 shown in the figure, debris 352 having an area intersects a spot boundary 351 whereas debris 353 having no area intersects two spot boundaries 351. Debris 354 also having no area exists in an area enclosed by a spot boundary 351. Debris 355 having an area exists inside an area enclosed by a spot boundary 351. Debris 356 having an area exists outside an area enclosed by a spot boundary 351. Debris 357 having no area also exists outside an area enclosed by a spot boundary 351. Debris 358 also having an area has the shape of a tear resembling a fluorescent sample flowing out off a spot. The debris 356, the debris 357 and the debris 358 are each debris located on the background image. Debris 360 is spread over 2 spots. To put it concretely, the debris 362 covers areas enclosed by spot boundaries 361 and 362.

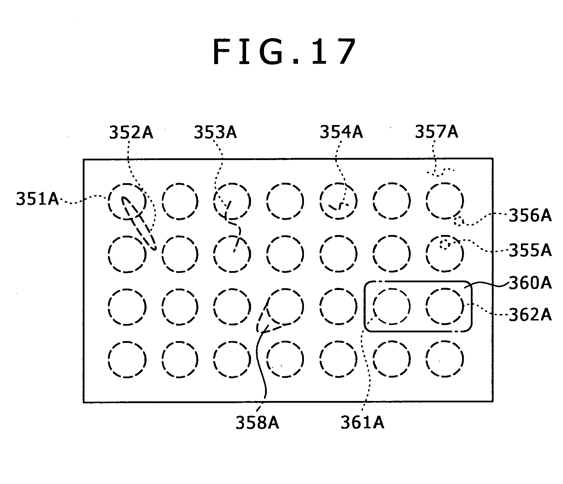

Fig. 17 is a diagram showing a model of a binary-coded image obtained as a result of a binary conversion process carried out on the basis of a threshold value on data resulting from a data differentiation process carried out on the image shown in Fig. 16. A spot boundary 351A, various kinds of debris 352A to 358A and 360A as well as spot boundaries 361A and 362A respectively corresponding to the spot boundary 351, the various kinds of debris 352 to 358 and 360 as well as the spot boundaries 361 and 362, which are shown in Fig. 16.

Then, at the next step S113, the debris-boundary extraction unit 323 carries out processing to extract debris boundaries. Details of the processing to extract debris boundaries will be explained later by referring to a flowchart shown in Fig. 19. By carrying out this processing, the boundary line of every piece of existing debris can be detected with a high degree of reliability for each pixel.

Then, at the next step S114, the debris-area elimination unit 324 carries out a process to eliminate each debris area intersecting a spot area. To put it in detail, a debris boundary detected in the processing carried out at the step S113 is compared with a spot area in order to determine each debris area intersecting a spot area as a debris area to be eliminated. For example, the pieces of debris 352, 353 and 360 shown in Fig. 16 are eliminated.

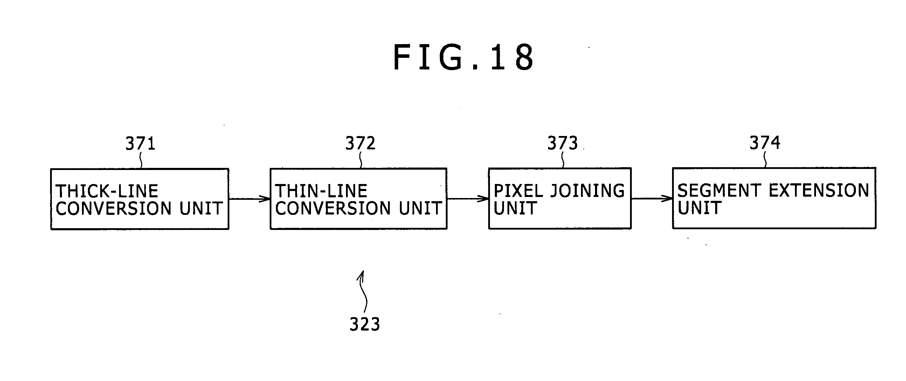

By referring to the flowchart shown in Fig. 19, the following description explains details of the processing carried out at the step S113 of the flowchart shown in Fig. 15 as processing to extract debris boundaries. In order for the debris-boundary extraction unit 323 shown in Fig. 14 to carry out this processing, as shown in Fig. 18, the debris-boundary extraction unit 323 is provided with a thick-line conversion unit 371, a thin-line conversion unit 372, a pixel joining unit 373 and a segment extension unit 374.

The thick-line conversion unit 371 is a section for converting the line of each pixel into a thick line. The thin-line conversion unit 372 is a section for converting a thickened line into a thin line. The pixel joining unit 373 is a section for joining pixels. The segment extension unit 374 is a section for extending a segment.

The processing carried out to extract debris boundaries is represented by the flowchart shown in Fig. 19. As shown in the figure, the flowchart begins with a step S151 at which the thick-line conversion unit 371 carries out a process to convert the line of each pixel into a thick line. To put it concretely, as shown in Fig. 20, the line of a pixel 401 is extended toward the surroundings to become a thick line 402.

Then, at the next step S152, the thin-line conversion unit 372 converts the thickened line into a thin line. Subsequently, at the next step S153, the pixel joining unit 373 carries out a process to join pixels. By converting the thickened line into a thin line and carrying out a process to join pixels, as shown in Fig. 21, an isolated pixel like the one shown in Fig. 20 is eliminated and a line inside the thick line 402 obtained as a result of the line thickening process carried out at the step S151 is converted into one thin line 411.

Then, at the next step S154, the segment extension unit 374 extends a segment. For example, as shown in Fig. 22, if the end point 421A of a segment 421 formed by a thin line and the end point 422A of a segment 422 formed by another thin line exist, a scanning operation is carried out in a predetermined scanning range 423 with the end point 421A taken as the center. Then, the scanning range 423 and the position of the end point 422A are examined in order to produce a result of determination as to whether or not the scanning range 423 includes the position of the end point 422A. If the result of the determination indicates that the scanning range 423 includes the position of the end point 422A, the end point 421A is extended to the end point 422A found in the search operation in order to concatenate the segments.

Then, if necessary, at the next step S155, a process to convert each debris boundary into a thick line is carried out.

Fig. 23 is an explanatory diagram to be referred to in description of a state in which the boundaries of pieces of debris 352A to 358A and 360A shown in Fig. 17 have each been made a thick line to result in debris boundaries 352B to 358B and 360B respectively in the process carried out at the step S155 of the flowchart shown in Fig. 19.

After the processing carried out at the step S113 of the flowchart shown in Fig. 15 has been completed, as described above, at the next step S114, the debris-area elimination unit 324 eliminates each debris area that intersects a spot area. The processing to eliminate each debris area that intersects a spot area is explained by referring to Fig. 24 as follows.

In a typical spot shown in Fig. 24, a debris area 352D is spread across a spot boundary 351 enclosing a spot area 351D of the spot. A vector 355 represented by an arrow shown in Fig. 24 is a vector perpendicular to the debris boundary 352B of the debris area 352D. Fig. 25 is a diagram showing a graph representing changes seen along the horizontal axis parallel to the direction of the vector 355 as changes of the fluorescence intensity represented by the vertical axis. As shown in the figure, data in the range of the debris area 352D is erased in the process carried out at the step S114. To put it more accurately, data in the range of the spot area 351D in close proximity to the debris boundary 352B is also erased as well. (The range of the spot area 351D in close proximity to the debris boundary 352B is a range of a predetermined distance from the debris boundary 352B.)

Fig. 26 is a block diagram showing a typical functional configuration of the hybridization-level computation unit 85 for carrying out processing at the step S38 of the flowchart shown in Fig. 5 to compute a hybridization level and a reliability. As shown in the figure, the hybridization-level computation unit 85 employs a spot division unit 441, an information computation unit 442, an area selection unit 443 and an output unit 444.

Functioning as division means, the area division unit 441 divides the area inside a spot into a plurality of spot internal areas each including or not including an unnecessary material. Functioning as computation means, the information computation unit 442 computes a reactivity and a reliability for every spot internal area. The reactivity is information representing the state a bioreaction undergone by a first biological substance in conjunction with a second biological substance in a reaction area. The reliability is the reliability of the reactivity. To put it concretely, the information computation unit 442 computes a hybridization value and a reliability for every spot internal area. In addition, the information computation unit 442 combines spot internal areas each included in one of a plurality of reaction areas used in measurements carried out under the same condition to form a combination across the reaction areas. Then, the information computation unit 442 computes a combination reactivity and a combination reliability for every combination. The combination reactivity is the reactivity of a combination whereas the combination reliability is the reliability of a combination. The combination reactivity of a combination having the largest combination reliability among the combination reliabilities of combinations across the reaction areas is taken as the reactivity of the reaction areas. The area selection unit 443 is a section for selecting a spot internal area. The output unit 444 is a section for outputting a hybridization value and a reliability for every spot.

By referring to a flowchart shown in Fig. 27, the following description explains details of the processing carried out at the step S38 of the flowchart shown in Fig. 5 as processing to compute a hybridization value and a reliability.

The flowchart shown in Fig. 27 begins with a step S201 at which the area division unit 441 divides the area of a spot into spot internal areas. To be more specific, the area division unit 441 divides the area inside a spot into a plurality of spot internal areas each including or not including debris. As an example, let us take a spot area 462 enclosed in a spot boundary 461 as shown in Fig. 28. The spot area 462 includes debris 464 and debris 465, which both do not have an area, and debris 463 having an area. Since the debris 464 and the debris 465 both do not have an area, it is obvious that they are each debris. Thus, their data is deleted at this stage.

On the other hand, the debris 463 is located inside the spot area 462 enclosed in the spot boundary 461 and does not intersect the spot boundary 461. Thus, in the process carried out at the step S114 of the flowchart shown in Fig. 15, data of the debris 463 is not erased.

A determination process explained below is a process to produce a result of determination as to whether any arbitrary observed point inside the area of an observed spot is a point inside or outside a debris area. To put it in detail, if a debris boundary has an intersection point with the outermost boundary line of a background image or has no such an intersection point, first of all, a straight line is drawn from any arbitrary point located on the outermost boundary line of the background image as a point other than a point on a debris boundary to any arbitrary observed point located in the area inside the observed spot as an observed point to be checked. Then, the number of intersection points of debris boundaries and the straight line is counted. If the number of intersection points is even, the observed point is determined to be a point outside a debris area. If the number of intersection points is odd, on the other hand, the observed point is determined to be a point inside a debris area.

That is to say, the area division unit 441 draws a straight line from an external point determined in advance to an observed point. Then, the area division unit 441 counts the number of such intersection points and when the number of such intersection points is odd, the observed point is a point inside a debris area. If the straight line is tangent to a debris boundary, however, the tangent point is counted as a point of intersection.

For example, in a case shown in Fig. 29, if a straight line 503 is drawn from a point 502 located on the upper side of the outermost boundary line 501 on the left side of the background image to an observed point 483A in the area of an observed spot 483, the straight line 503 intersects the debris boundary 482A of debris 482 and the debris boundary 481A of debris 481. To be more specific, the straight line 503 intersects the debris boundary 482A of the debris 482 at four intersection points 491, 492, 493 and 494 as well as intersects the debris boundary 481A of the debris 481 at an intersection point 495. Thus, the straight line 503 intersects five intersection points. Since the number of intersection points is 5, which is an odd number, the observed point 483A is determined to be a point inside the debris 481.

Fig. 30 is a diagram showing a typical case in which the area of a spot is divided into spot internal areas. As shown in the figure, a spot area 462 having a spot boundary 461 as shown in Fig. 28 is divided into a spot internal area 522 including debris 463 and a spot internal area 521 including no debris.

Then, at the next step S202, the information computation unit 442 computes a hybridization value and a reliability for each spot internal area. The hybridization value $ah_i^j$ (or the reactivity) of a spot internal area i and the reliability $ar_i^j$ of the spot internal area i are expressed by the following equations respectively:

[Formula 2]

$$\begin{cases} ah_i^{\,j} = \dfrac{1}{pn_i^{\,j}} \sum_{k=1}^{pn_i^{\,j}} hybridization(pf_i^{\,jk}) & ...(4) \\[4mm] ar_i^{\,j} = \dfrac{1}{pn_i^{\,j}} \sum_{k=1}^{pn_i^{\,j}} confidence(pf_i^{\,jk}) & ...(5) \end{cases}$$

In Eqs. (4) and (5), notation i denotes the number of a spot, notation j denotes the number of a spot internal area in a spot i and notation k denotes the number of a pixel in a spot internal area.

In Eq. (4), notation hybridization $(pf_i^{jk})$ denotes values given by equations represented by the curves 191 to 194 shown in Fig. 7 whereas notation $pn_i^j$ denotes the number of pixels in a spot area j.

Notation confidence $(pf_i^{jk})$ used in Eq. (5) denotes a quantity expressed by Eq. (6) as follows:

[Formula 3]

$$confidence(pf) = w_c \times w_{sat} \times variance(pf)^{-1} \qquad ...(6)$$

Notation $w_c$ used in Eq. (6) denotes a weight coefficient of high-reliability segments, which are segments outside the segments 191A to 194A and 191B to 194B of respectively the curves 191 to 194 shown in Fig. 7, whereas notation

$w_{sat}$ denotes a weight coefficient of low-reliability segments, which are the segments 191A to 194A and 191B to 194B of respectively the curves 191 to 194.

Fig. 31 is a diagram showing a coordinate space in which there are plotted points 701 each representing a pixel in a spot. The horizontal coordinate of each point 701 is the result of a first measurement of the fluorescence intensity of the point 701 whereas the vertical coordinate of the point 701 is the result of a second measurement of the fluorescence intensity of the point 701. As shown in the figure, point positions each prescribed by fluorescence intensities obtained as results of the first and second measurements for every point 701 as the position of the point 701 are spread in the vicinity of a straight line 702 having a gradient of 45 degrees. Ideally, the first and second measurements produce the same fluorescence intensity for the same pixel in a spot. Thus, the points 701 should be ideally located on the straight line 702. In actuality, however, the fluorescence intensity varies from measurement to measurement. Thus, the points 701 are never perfectly located on the straight line 702. Instead, the points 701 are spread in the vicinity of the straight line 702. There is a tendency of the measurement results to have large spreading variations for relatively small fluorescence intensities and small spreading variations for relatively large fluorescence intensities. To put it concretely, in a range 703 set for small fluorescence intensities $ph_1$, the variances are relatively large. In a range 703 set for fluorescence intensities $ph_2$ larger than fluorescence intensities $ph_1$, the variances are smaller than the variances for the fluorescence intensities $ph_1$. Likewise, in a range 703 set for fluorescence intensities $ph_3$ even larger than fluorescence intensities $ph_2$, the variances are even smaller than the variances for the fluorescence intensities $ph_2$. Notation variance (pf) used in Eq. (6) denotes a variance computed for points 701 inside a predetermined range 703 set in advance. To put it in detail, confidence (pf) used in Eq. (6) as a notation denoting a confidence level is proportional to the reciprocal of the variance (pf) of points each included in a range as a point representing a fluorescence intensity obtained as a result of a hybridization undergone by two biological substances used as first and second biological substances in a first measurement and representing a fluorescence intensity obtained as a result of a hybridization undergone by the same 2 biological substances in a second measurement. That is to say, the confidence (pf) is proportional to 1/variance (pf).

Thus, the confidence (pf) in Eq. (6) can also be computed from stored data obtained as a result of measurements carried out in the past. Therefore, as an asset obtained in the past, a fluorescent image can be evaluated and utilized effectively. This also means that the processing to take a fluorescent image can be carried out by making use of an apparatus other than an apparatus used for evaluating the fluorescent image, and the fluorescent image can be evaluated at a time different from the time at which the fluorescent image is taken. In addition, in order to take a fluorescent image and evaluate the fluorescent image, it is not necessary to take a special image other than the fluorescent image. It is thus possible to prevent the apparatus from becoming complicated, the manufacturing cost of the apparatus from increasing, the number of processing steps from rising and the processing from becoming complex.

Let us refer back to Fig. 27. At the next step S203, the area selection unit 443 selects a spot internal area.

In order to carry out the aforementioned processing to select a spot internal area, the area selection unit 443 shown in Fig. 26 needs to be designed into a functional configuration like one shown in Fig. 32. As shown in Fig. 32, the area selection unit 443 employs a reliability computation unit 721, a reliability determination unit 722 and a flag setting unit 723.

The reliability computation unit 721 is a section for correcting a reliability of a spot internal area on the basis of a spot reliability. The reliability determination unit 722 is a section for carrying out processing such as comparison of a corrected reliability of a spot internal area with a threshold value and determination based on a result of reliability determination by mechanical learning. Functioning as flag setting means, the flag setting unit 723 is a section for setting a flag based on reliability as a flag indicating whether or not a reactivity can be used. If the reaction area is divided into a partial area including an unnecessary substance and a partial area including no unnecessary substance, the flag is set in on the basis of the number of partial areas or a command entered by the operator.

The processing carried out by the area selection unit 443 to select a spot internal area is explained by referring to a flowchart shown in Fig. 33.

As shown in the figure, the flowchart begins with a step S251 at which the reliability computation unit 721 computes the product of the reliability of a spot internal area and the spot reliability. The reliability of a spot internal area is denoted by notation $ar_j^i$ in Eq. (5) whereas the spot reliability is a quantity expressed by Eq. (7) as follows:

[Formula 4]

Spot reliability = 1 - (Average of spot peripheral

```
fluorescence intensities / Average of spot fluorescence

intensities)  …  (7)
```

As an example, let us assume that that a spot 738 shown in Fig. 34 is an observed spot. In this case, the average value of spot fluorescence quantities (that is, the denominator of an expression on the right side of Eq. 7) is the average value of pixel values of pixels composing the spot 738 whereas the average value of spot peripheral fluorescence intensities (that is, the numerator of the expression on the right side of Eq. 7) is the average value of pixel values of pixels forming the spot peripheral area 739 of the spot 738. The spot peripheral area 739 is a periphery surrounding the spot 738. (In this example, the spot peripheral area has a width equal to about 1/2 times the distance between the spot boundary of the spot 738 and the spot boundary of a spot, which is a spot adjacent to the spot 738.)

The smaller the average value of spot peripheral fluorescence intensities and the larger the average value of spot fluorescence quantities, the larger the spot reliability expressed by Eq. (7) or the closer the spot reliability to 1.

Thus, by multiplying the reliability $ar_j$ expressed by Eq. (5) as the reliability of the spot internal area by the spot reliability, the reliability of the spot internal area can be corrected.

Then, at the next step S252, the reliability determination unit 722 produces a result of determination as to whether or not the reliability corrected in the process carried out at the step S251 as the reliability of the spot internal area is smaller than a threshold value. If the determination result produced in the process carried out at the step S252 indicates that the corrected reliability of the spot internal area is not smaller than the threshold value (that is, the corrected reliability of the spot internal area is equal to or greater than the threshold value), the flow of the processing represented by this flowchart goes on to a step S253 at which the reliability determination unit 722 produces a result of determination as to whether or not a spot area has been used in reliability determination based on mechanical learning. As will be explained later by referring to Fig. 40, the reliability determination based on mechanical learning is typically reliability determination making use of a SVM (Support Vector Machine). If the corrected reliability of the spot area is determined to be small even for use of the SVM in reliability determination (that is, if a scrapping flag based on the reliability of the data of the spot area has been set to indicate that the spot area was not used in reliability determination based on mechanical learning), the flow of the processing represented by this flowchart goes on to a step S254 at which the flag setting unit 723 sets a scrapping flag based on the reliability of the data of the spot internal area. That is to say, in this case, the data of the observed spot internal area is not reliable. Thus, basically, the data of the observed spot internal area is not used thereafter.

If the determination result produced in the process carried out at the step S252 indicates that the corrected reliability of the spot internal area is smaller than the threshold value, on the other hand, the flow of the processing represented by this flowchart skips the process to produce a result of determination as to whether or not the spot area has been used in reliability determination based on mechanical learning. That is to say, since the data of the spot internal area is obviously unreliable in this case, the flow of the processing represented by this flowchart goes directly to the step S254 at which the flag setting unit 723 sets the scrapping flag based on the reliability of the data of the spot internal area.

If the determination result produced in the process carried out at the step S253 indicates that the spot area has been used in reliability determination based on mechanical learning (that is, if the scrapping flag based on the reliability of the data of the spot area is not set), on the other hand, the flow of the processing represented by this flowchart skips the process carried out at the step S254 to set the scrapping flag based on the reliability of the data of the spot internal area. That is, the scrapping flag based on the reliablity is not set to the spot internal area.

In actuality, an operation to set the scrapping flag based on the reliability of the data of a spot internal area as described above is not an operation to really scrap the data of the spot internal area itself. Instead, the scrapping flag is merely set in the data of each spot internal area. In this way, the scrapping flag can of course be utilized to indicate that the data of the spot internal area is to be excluded if necessary from use in subsequent processes. Conversely, the data of the spot internal area can be used in subsequent processes by deliberately ignoring the scrapping flag.

It is to be noted that in the process carried out at the step S203 to select a spot internal area, the user is also allowed to carry out a manual operation to set the scrapping flag in the data of the spot internal area (that is, the user is also allowed to enter an input indicating whether or not the scrapping flag is to be set). In the case of typical spots shown in Fig. 34, for example, a portion of the debris boundary 736 of a debris area 735 overlaps a portion of the spot boundary 732 of a spot 731. The debris area 735 covers the entire spot 731 and the entire area of a spot 733 located at a position adjacent to the spot 731 on the right side of the figure as a spot including a spot internal area 734. With the images of the spots 731 and 733 displayed on the display unit 29A, the user is capable of setting the scrapping flags based on the reliabilities of the data of the spot internal areas in the spots 731 and 732 for the spot internal areas by operating the UI unit 29.

Let us refer back to the flowchart shown in Fig. 27. As described above, after the process carried out at the step S203 to select a spot internal area has been completed, the flow of the processing represented by the flowchart shown in the figure goes on to a step S204 at which the output unit 444 shown in Fig. 26 carries out processing to output a hybridization value and a reliability for each spot. In order for the output unit 444 to carry out the processing to output a hybridization value and a reliability for each spot, the output unit 444 shown in Fig. 26 is provided with a functional configuration like one shown in Fig. 35. The output unit 444 shown in Fig. 35 employs a spot-count determination unit 751, a combination generation unit 752, a computation unit 753, a combination selection unit 754 and a flag setting unit 755.

The spot-count determination unit 751 is a section for comparing the number of divided spots with a threshold value. The combination generation unit 752 is a section for generating a combination of spot internal areas. The computation unit 753 is a section for computing a hybridization level and a reliability for each spot. The combination selection unit 754 is a section for selecting a combination having the highest reliability. The flag setting unit 755 is a section for setting a flag.

By referring to a flowchart shown in Fig. 36, the following description explains the processing carried out by the output unit 444 to output a hybridization value and a reliability for each spot.

As shown in the figure, the flowchart begins with a step S301 at which the spot-count determination unit 751 produces a result of determination as to whether or not the number of divided spots is smaller than a predetermined threshold value set in advance. That is to say, the number of specific spots each divided into spot internal areas is counted. The specific spots each divided into spot internal areas are among a plurality of existing spots. As described earlier, the area of each specific spot is divided into spot internal area in the process carried out at the step S201 of the flowchart shown in Fig. 27.

If the determination result produced in the process carried out at the step S301 indicates that the number of divided spots is smaller than the predetermined threshold value, the flow of the processing represented by this flowchart goes on to a step S302 at which the combination generation unit 752 generates combinations of spot internal areas. Fig. 37 is an explanatory diagram to be referred to in description of the combinations. As shown in the figure, let us assume that n experiments have been carried out on respectively n substrates. That is to say, any one of the experiments has been carried out on one of the substrates. In other words, in the case of the substrates shown in Fig. 37, the first experiment has been carried out on the substrate $11A_1$, the second experiment has been carried out on the substrate $11A_2$ and so on. Finally, the nth experiment has been carried out on the substrate $11A_n$. A probe is immobilized for spots at positions of the same coordinates on the substrates. It is to be noted that, instead of having spots located at positions of the same coordinates on different substrates subjected to different experiments respectively as described above, in actuality, the spots located at positions of the same coordinates on different substrates, which are merely subjected to the same experiment, or each spot on the same substrate can be merely subjected to different experiments.

For example, a spot $771_1$ exists on the right-upper corner of the substrate $11A_1$. By the same token, a spot $771_2$ exists on the right-upper corner of the substrate $11A_2$. In the same way, a spot $771_n$ exists on the right-upper corner of the substrate $11A_n$. These spots are the aforementioned spots existing at positions having the same coordinates. The spot $771_1$ on the substrate $11A_1$ is divided into three spot internal areas each denoted by the character R in Fig. 37. The number appended as a lower-side suffix to the character R indicates the substrate (or the experiment). On the other hand, the number appended as an upper-side suffix to the character R is the number assigned to the spot internal area (or a partial area obtained as a result of dividing a spot) denoted by the character R. Thus, the spot $771_1$ existing is divided into 3 spot internal areas $R_1^1$, $R_1^2$ and $R_1^3$. The hybridization level (or the hybridization value expressed by Eq. (4)) of the spot internal area $R_1^1$ is denoted by notation $ah_1^1$. By the same token, the hybridization level of the spot internal area $R_1^2$ is denoted by notation $ah_1^2$. In the same way, the hybridization level of the spot internal area $R_1^3$ is denoted by notation $ah_1^3$. On the other hand, the spot $771_2$ existing on the substrate $11A_2$ is handled as one spot internal area $R_2^1$ and the hybridization level of the spot internal area $R_2^1$ is denoted by notation $ah_2^1$. The spot $771_n$ existing on the substrate $11A_n$ subjected to the nth experiment is divided into two spot internal areas $R_n^1$ and $R_n^2$. The hybridization level of the spot internal area $R_n^1$ is denoted by notation $ah_n^1$ whereas the hybridization level of the spot internal area $R_n^2$ is denoted by notation $ah_n^2$.

In a process to combine spot internal areas, a spot internal area is selected from each substrate. For example, as shown in Fig. 38, a spot internal area is selected from the three spot internal areas $R_1^1$, $R_1^2$ and $R_1^3$ of the spot $771_1$. The spot internal area $R_2^1$ is selected since the spot internal area $R_2^1$ is the only spot internal area included in the spot $771_2$. A spot internal area is selected from the two spot internal areas $R_n^1$ and $R_n^2$ of the spot $771_n$. As a result, for n = 3, there are six (= 3 × 1×2) possible combinations.

To put it concretely, the six combinations of spot internal areas are $R_1^1$-$R_2^1$-$R_3^1$, $R_1^2$-$R_2^1$-$R_3^1$, $R_1^3$-$R_2^1$-$R_3^1$, $R_1^1$-$R_2^1$-$R_3^2$, $R_1^2$-$R_2^1$-$R_3^2$ and $R_1^3$-$R_2^1$-$R_3^2$.

Then, at the next step S303 of the flowchart shown in Fig. 36, the computation unit 753 computes a hybridization level and a reliability for each spot. To put it concretely, the following Eqs. (8) and (9) are computed:

[Formula 5]

$$Sh = \frac{1}{Sn} \sum_{i=1}^{n} ah_i \cdot pn_i \qquad ...(8)$$

$$Sr = \frac{1}{Sn} \sum_{i=1}^{n} ar_i \cdot pn_i \qquad ...(9)$$

$$Sn = \sum_{i=1}^{n} pn_i \qquad ...(10)$$

Notation i used in Eqs. (8) to (10) denotes the number of times the experiment is carried out. That is to say, notation i (i = 1, 2, ... n) is a number assigned to an experiment carried out on one of the substrates shown in Fig. 37.

Notation $pn_i$ denotes the number of pixels in a spot internal area included in a combination as a spot internal area subjected to the ith experiment. Notation $S_n$ denotes the total number of pixels in spot internal areas included in the combination. For example, $S_n$ is the total number of pixels in the spot internal areas $R_1^1$, $R_2^1$ and $R_3^1$.

Notation $ah_i$ used in Eq. (8) denotes the hybridization level of a spot internal area included in the combination as a spot internal area subjected to the ith experiment. To put it concretely, the hybridization level is the hybridization value expressed by Eq. (4). On the other hand, notation $ar_i$ used in Eq. (9) denotes the reactivity of a spot internal area included in the combination as a spot internal area subjected to the ith experiment. To put it concretely, the reliability ari is the reliability expressed by Eq. (5).

Thus, notation Sh used in Eq. (8) denotes the hybridization level of the combination whereas notation Sr used in Eq. (9) denotes the reliability of the combination.

Then, at the next step S304 of the flowchart shown in Fig. 36, the combination selection unit 754 selects a combination providing the highest reliability (or the smallest variance) among combinations. That is to say, the process is carried out at the step S303 to compute a reliability Sr for each of possible combinations and a combination providing the highest reliability among all the possible combinations is selected.

In the typical case shown in Fig. 38, for example, there are six possible combinations. In this case, reliabilities $Sr_1$ to $Sr_6$ are computed for the combinations $R_1^1$-$R_2^1$-$R_3^1$ to $R_1^3$-$R_2^1$-$R_3^2$ respectively and a combination having the highest reliability is then selected. Let us assume for example that the reliability $Sr_1$ is the highest reliability. In this case, the combination $R_1^1$-$R_2^1$-$R_3^1$ is selected.

If the determination result produced in the process carried out at the step S301 indicates that the number of divided spots is equal to or greater than the predetermined threshold value, on the other hand, the flow of the processing represented by this flowchart skips the processes of the steps S302 to S304. At the step S305, the flag setting unit 755 sets a scrapping flag based on the reliability.

Let us assume that n2 spots of the n spots $771_1$ to $771_n$ shown in Fig. 37 have each been divided into spot internal areas and $n_2$ is greater than the predetermined threshold value $n_1$. In this case, it is possible to assume that most of the n spots $771_1$ to $771_n$ are each a spot including debris. It is thus quite within the bounds of possibility that the measurement environment itself has some problems. In such a case, the reliability of the data of the spots themselves can be considered to have been lost. Thus, the scrapping flag based on reliability as the flag of each of the spots is set.

Fig. 39 is a diagram showing the format of expression profile data supplied by the output unit 146 to the storage unit 147 (the expression profile data storage unit 28 in Fig. 1) in the experimental-process processing apparatus shown in Fig. 3 in the process carried out at the step S16 of the flowchart shown in Fig. 4. As shown in Fig. 39, the expression profile data 801 includes a spot count N, a repeated-experiment count n and data for combinations. If the number of spots for a probe in an experiment is one, the spot count N is equal to the repeated-experiment count n. The format of the expression profile data 801 shows data for m combinations, i. e., the first to mth combinations. The data for the m combinations includes hybridization levels $Sh_1$ to $Sh_m$ for the first to mth combinations respectively, reliabilities $Sr_1$ to $Sr_m$ for the first to mth combinations respectively, scrapping flags $f_1$ to $f_m$ for the first to mth combinations respectively and pointers $P_1$ to $P_m$ for the first to mth combinations respectively. For example, the pointer $P_1$ is a pointer for the first combination. The pointer $P_1$ points to a position used for storing the link data 802 for the first combination. The link data 802 includes the spot number N and pointers $P_{11}$ to $P_{1N}$ each pointing to the data of one of the first to Nth spots.

For example, the pointer $P_{11}$ is a pointer pointing to a position used for storing the spot data of the first spot composing the first combination.

The spot data 803 of a spot includes a spot-internal-area count an representing the number of spot internal areas composing the spot, a spot internal area number 'selected-no', which is a number assigned to a spot internal area selected for the combination and a spot reliability. In this case, the spot reliability is the quantity expressed by Eq. (7).

For each of spot internal areas composing a spot, the spot data 803 for the spot further includes a hybridization level ah, a reliability ar, a pixel count pn and a scrapping flag f. The spot data 803 shown in Fig. 39 for the first spot includes hybridization levels $ah_1{}^1, ar_1{}^2, ah_1{}^3$ for respectively the first to third spot internal areas of the first spot, reliabilities $ar_1{}^1, ar_1{}^2, ar_1{}^3$ for respectively the first to third spot internal areas of the first spot, pixel counts $pn_1{}^1, pn_1{}^2, pn_1{}^3$ for respectively the first to third spot internal areas of the first spot and scrapping flags $f_1{}^1, f_1{}^2, f_1{}^3$ for respectively the first to third spot internal areas of the first spot.

Fig. 40 is an explanatory diagram referred to in description of the principle of reliability determination processing carried out at the step S253 of the flowchart shown in Fig. 33 on the basis of a mechanical learning process.

In the learning mode, the user supplies the expression profile data 801, which includes the link data 802 and the spot data 803, to an SVM 91. The user also makes a request to display the image of each spot associated with the expression profile data 801 on the display unit 29A employed in the UI unit 29. Then, the user operates the UI unit 29 in order to enter a command to take each of the spots as a used spot 822 or an unused spot 823. An unused spot 823 means a spot, which is determined by the user to be a spot not to be used due to existence of debris or the like in the spot after visual confirmation. On the other hand, a used spot means a spot, which is determined by the user to be a spot at which a valid hybridization has been activated due to inexistence of debris or the like. In typical expression profile data 821 shown in Fig. 40, a spot marked with an X symbol is an unused spot 823 and a spot not marked with an X symbol is a used spot 822.

A command entered by the user to the SVM 91 as a command to handle any arbitrary spot as a used spot or an unused spot means a command indicating teaching data to be used as the scrapping flags $f_1$ to $f_m$ included in the expression profile data 801 as flags for each spot.

The SVM 91 carries out a process to learn relations between the teaching data indicated by the user to be used as the scrapping flags $f_1$ to $f_m$ and information included in the expression profile data 801 as learning data such as a hybridization level ah for each spot and a reliability ar for each spot. The SVM 91 then stores results of the learning process in a spot elimination pattern DB 92.

By carrying out a learning process on a number of spots in a learning mode as described above, it is possible to construct a spot elimination pattern DB 92 in advance and supply expression profile data 841 having data of spots 842 to the SVM 91 and request the SVM 91 to determine whether or not to use data included in the expression profile data 841 as the data of the spots 842 by referring to the spot elimination pattern DB 92 in a determination mode as shown in Fig. 41.

That is to say, the SVM 91 refers to the spot elimination pattern DB 92 and takes each of the spots 842 as a used spot 842A or an unused spot 842B on the basis of the data included in the expression profile data 841 as the data of the spots 842. In the typical expression profile data 841 shown in Fig. 41, a spot marked with an X symbol is a spot determined to be an unused spot 842B and a spot not marked with an X symbol is a spot determined to be a used spot 842A.

As described above, the scrapping flags $f_1$ to $f_m$ included in the expression profile data 801 as flags for each spot are set in a learning process. However, the scrapping flags $f_i{}^j$ for spot internal areas can also be set in a learning process.

It is to be noted that details of the SVM are described in a Cambridge publication authored by Nello Cristianini and John Shawe-Taylor with a title of "An Introduction to Support Vector Machines and Other Kernel-Based Learning Methods."

The determination result produced in the process carried out at the step S253 of the flowchart shown in Fig. 33 is TRUE if a spot (or a spot internal area) is determined to be a spot (or a spot internal area) to be used or FAULT if a spot (or a spot internal area) is determined to be a spot (or a spot internal area) not to be used. As described before, if the result of the determination is FAULT, the flag setting unit 723 sets a scrapping flag based on the reliability of the data of the spot internal area.

It is to be noted that, in a learning process, a neural network can be typically used as a substitute for the SVM.

The above description explains an embodiment for a case in which hybridization of a DNA chip is measured. However, the scope of the present invention is by no means limited to a DNA chip. That is to say, the present invention can be applied to a case in which it is necessary to carry out a measurement in order to determine whether or not a biological substance having one of a variety of types has been biologically joined to another biological substance determined in advance.

The series of processes described previously can be carried out by hardware and/or execution of software. Typically, the biological-information processing apparatus 1 is implemented as a personal computer 901 like one shown in Fig. 42.

In the personal computer 901 shown in Fig. 42, a CPU (Central Processing Unit) 921 carries out various kinds of processing by execution of programs stored in a ROM (Read Only Memory) 922 or programs loaded from a storage

## EP 1 873 514 A1

section 928 into a RAM (Random Access Memory) 923. The RAM 923 is also used for properly storing various kinds of information such as data required in execution of the processing.

The CPU 921, the ROM 922 and the RAM 923 are connected to each other by a bus 924, which is also connected to an input/output interface 925.

The input/output interface 925 is connected to an input section 926, an output section 927, the storage section 928 cited above and a communication section 929. The input section 926 includes a keyboard and a mouse whereas the output section 927 includes a display unit and a speaker. The display unit can be a CRT (Cathode Ray Tube) display unit or an LCD (Liquid Crystal Display) unit. The storage section 928 includes a hard disk. The communication section 929 has a modem. The communication section 929 is a unit for carrying out communication processing with other apparatus through a network including the Internet.

The input/output interface 925 is also connected to a drive 930 on which a removable recording medium 931 is mounted. The removable recording medium 931 can be a magnetic disk, an optical disk, a magneto-optical disk or a semiconductor memory. As described above, a computer program is installed from the removable recording medium 931 into the storage section 928 as occasions demand.

As explained earlier, the series of processes described previously can be carried out by hardware and/or execution of software. If the series of processes described above is carried out by execution of software, programs composing the software can be installed into a computer embedded in dedicated hardware, a general-purpose personal computer or the like from typically a network or a recording medium. A general-purpose personal computer is a personal computer, which can be made capable of carrying out a variety of functions by installing a variety of programs into the personal computer.

The aforementioned recording medium is the removable recording medium 931 provided to the user separately from the main unit of the personal computer as shown in Fig. 42. Examples of the removable recording medium 931 include the magnetic disk such as a flexible disk, the optical disk such as a CD-ROM (Compact Disk - Read Only Memory) or a DVD (Digital Versatile Disk), the magneto-optical disk such as an MD (Mini Disk) as well as the semiconductor memory. Instead of installing the programs from the removable recording medium 931, the programs to be provided to the user can also be stored in advance in an embedded recording medium included in the main unit. Examples of the embedded recording medium are a hard disk included in the storage section 928 and the ROM 922.

It is also worth noting that, in this description, steps of the flowcharts described above can of course be carried out in a pre-prescribed order along the time axis. However, the steps of the flowcharts described above can be carried out not only in a pre-prescribed order along the time axis, the but also concurrently or individually.

It is also to be noted that the technical term 'system' used in this specification implies the configuration of a confluence including a plurality of apparatus or a plurality of functional modules each used for executing a specific function. The apparatus or the functional modules are logically connected to each other and do not have to be incorporated in the same physical case.

**Claims**

1. A biological-information processing apparatus for measuring the state of a bioreaction undergone by a first biological substance immobilized in a reaction area provided on a substrate in conjunction with a second biological substance biologically reacting to said first biological substance, said biological-information processing apparatus comprising:

    inputting means for inputting image information of said reaction area; and
    computation means for computing reactivity information representing said state of said bioreaction undergone by said first biological substance immobilized in said reaction area in conjunction with said second biological substance and computing reliability information representing the reliability of said reactivity on the basis of said image information input by said inputting means.

2. The biological-information processing apparatus according to claim 1, said biological-information processing apparatus further having setting means for setting a flag indicating whether or not said reactivity

3. The biological-information processing apparatus according to claim 2 wherein said setting means sets said flag on the basis of said reliability information, the number of partial areas obtained as a result of dividing said reaction area into said partial areas each including an unnecessary substance and said partial areas each including no unnecessary substance and a command entered by an operator:

4. The biological-information processing apparatus according to claim 1, said biological-information processing apparatus further having reaction-division means for dividing said reaction area into partial areas each including an unnecessary substance and partial areas each including no unnecessary substance, wherein said computation means computes said reactivity information and said reliability information for each of said partial areas.

5. The biological-information processing apparatus according to claim 4 wherein:

partial areas each picked up from one of a plurality of said reaction areas used in measurements carried out under the same condition are combined to form a combination;
for each of such combinations, combination reactivity information used as reactivity information of said combination and combination reliability information used as reliability information of said combination are computed; and
said combination reactivity information of said combination having the highest combination reliability information among said combinations is used as said reactivity information of said reaction areas.

6. The biological-information processing apparatus according to claim 1 wherein:

said first biological substance and said second biological substance are genes having mutually complementary base sequences or each a substance derived from said gene; and
said reactivity information is hybridization information of said first biological substance and said second biological substance.

7. The biological-information processing apparatus according to claim 6 wherein said hybridization information is information determined unambiguously on the basis of a function from a fluorescence intensity obtained as a result of a hybridization undergone by said first biological substance in conjunction with said second biological substance.

8. The biological-information processing apparatus according to claim 7 wherein said reliability information is information proportional to the reciprocal of the variance of points each included in a range as a point representing a fluorescence intensity obtained as a result of a hybridization undergone by said first second biological substance in conjunction with said second biological substance in a first measurement and representing a fluorescence intensity obtained as a result of a hybridization undergone by said first second biological substance in conjunction with said second biological substance in a second measurement.

9. A biological-information processing method to be adopted in a biological-information processing apparatus for measuring the state of a bioreaction undergone by a first biological substance immobilized in a reaction area provided on a substrate in conjunction with a second biological substance biologically reacting to said first biological substance, said biological-information processing method comprising:

an inputting step of inputting image information of said reaction area; and
a computation step of computing reactivity information representing said state of said bioreaction undergone by said first biological substance immobilized in said reaction area in conjunction with said second biological substance and computing reliability information representing the reliability of said reactivity on the basis of said image information input at said inputting step.

10. A biological-information processing program of a biological-information processing apparatus for measuring the state of a bioreaction undergone by a first biological substance immobilized in a reaction area provided on a substrate in conjunction with a second biological substance biologically reacting to said first biological substance, said biological-information processing program executed by a computer to drive said biological-information processing apparatus to carry out:

an inputting step of inputting image information of said reaction area; and
a computation step of computing reactivity information representing said state of said bioreaction undergone by said first biological substance immobilized in said reaction area in conjunction with said second biological substance and computing reliability information representing the reliability of said reactivity on the basis of said image information input at said inputting step.

11. A recording medium used for recording a biological-information processing program according to claim 10.

# FIG.1

EP 1 873 514 A1

# FIG.2

EP 1 873 514 A1

# FIG. 3

# F I G . 4

```
( EXPERIMENTAL PROCESS PROCESSING START )
                      │
                      ▼
        ┌──────────────────────────────┐  S11
        │        PREPARE TARGET         │
        └──────────────────────────────┘
                      │
                      ▼
        ┌──────────────────────────────┐  S12
        │          HYBRIDIZE            │
        └──────────────────────────────┘
                      │
                      ▼
        ┌──────────────────────────────┐  S13
        │  ACQUIRE FLUORESCENCE INTENSITY │
        └──────────────────────────────┘
                      │
                      ▼
        ┌──────────────────────────────┐  S14
        ││   ESTIMATE EXPRESSION AMOUNT  │││
        └──────────────────────────────┘
                      │
                      ▼
        ┌──────────────────────────────┐  S15
        │        STANDARDIZE DATA        │
        └──────────────────────────────┘
                      │
                      ▼
        ┌──────────────────────────────┐  S16
        │  OUTPUT EXPRESSION PROFILE DATA │
        └──────────────────────────────┘
                      │
                      ▼
                  (   END   )
```

EP 1 873 514 A1

# F I G . 5

START OF EXPRESSION-AMOUNT
ESTIMATION PROCESSING

INPUT IMAGE INFORMATION — S31

IS EXCITATION
LIGHT INTENSITY INFORMATION
INCLUDED? — S32
TRUE
FAULT

ESTIMATE EXCITATION LIGHT INTENSITY — S33

IMAGE TAKEN
BY PLURALITY OF EXCITATION
LIGHT INTENSITIES? — S34
FAULT
TRUE

CREATE EQUATION FOR HYBRIDIZATION (pf)
USING PLURALITY OF EXCITATION LIGHT BEAMS — S35

PERFORM IMAGE PROCESSING — S36

VALIDATE HYBRIDIZATION — S37

COMPUTE HYBRIDIZATION
LEVEL AND RELIABILITY — S38

COMPUTE EXPRESSION AMOUNT — S39

RETURN

# F I G . 6

| THE NUMBER OF IMAGES FORMING A SET | | |
|---|---|---|
| EXCITATION LIGHT INTENSITY | THE NUMBER OF HORIZONTALLY AND VERTICALY ARRANGED PIXELS | FLUORESCENT IMAGE |
| ⋮ | ⋮ | ⋮ |

~181

| SPOT POSITION TEMPLATE IMAGE |
|---|
| THE UNMBER OF SPOTS |
| PROBE GENE INDEX |
| ⋮ |

~182

INPUT-IMAGE FORMAT

EP 1 873 514 A1

# F I G . 7

EP 1 873 514 A1

# FIG.8

211 — BACKGROUND-IMAGE SEPARATION UNIT

212 — NOISE ELIMINATION UNIT

213 — AREA ELIMINATION UNIT

214 — IMAGE DECOMPOSITION UNIT

83

EP 1 873 514 A1

# F I G . 9

```
┌─────────────────────────────────────────┐
│        IMAGE PROCESSING START            │
└─────────────────────────────────────────┘
                    │
                    ▼
┌─────────────────────────────────────────┐
│   SEPARATE BACKGROUND IMAGE          S61 │
│   BY USING TEMPLATE                      │
└─────────────────────────────────────────┘
                    │
                    ▼
┌─────────────────────────────────────────┐
│ ELIMINATE NOISES BY USING            S62 │
│ BACKGROUND-IMAGE CHARACTERISTICS         │
└─────────────────────────────────────────┘
                    │
                    ▼
┌─────────────────────────────────────────┐
│   ELIMINATE DEBRIS AREA SPREAD       S63 │
│   OVER SPOT BOUNDARY                     │
└─────────────────────────────────────────┘
                    │
                    ▼
┌─────────────────────────────────────────┐
│   DISASSEMBLE INPUT IMAGE            S64 │
│   INTO SPOT IMAGES                       │
└─────────────────────────────────────────┘
                    │
                    ▼
              ( RETURN )
```

# FIG.10

231                                        221

PARALLEL MOVEMENT,
ROTATION AND
ENLARGEMENT/
SHRINKING

232 222

221    231

# FIG.11

ENTIRE IMAGE

241 —| FREQUENCY FILTER |

242 —| TREND FILTER |

# FIG.12

222 221A

S71
TWO-DIMENSIONAL
FOURIER TRANSFORMATION

Power

261

FREQUENCY

S72
FILTER GENERATION

1

271

FREQUENCY

# FIG.13

EP 1 873 514 A1

EP 1 873 514 A1

# FIG.14

321  322  323  324

| DATA DIFFERENTIATION UNIT | → | BINARY-CONVERSION UNIT | → | DEBRIS-BOUNDARY EXTRACTION UNIT | → | DEBRIS-AREA ELIMINATION UNIT |

213

# FIG.15

START OF PROCESSING TO
ELIMINATE DEBRIS AREA
SPREAD OVER SPOT BOUNDARY

DIFFERENTIATE DATA
OF ENTIRE IMAGE | S111

PERFORM BINARY CONVERSION
USING THRESHOLD VALUE | S112

EXTRACT DEBRIS BOUNDARY | S113

ELIMINATE DEBRIS AREA
INTERSECTING SPOT AREA | S114

RETURN

# FIG.16

# FIG.17

# FIG.18

EP 1 873 514 A1

371

| THICK-LINE CONVERSION UNIT |

372

| THIN-LINE CONVERSION UNIT |

373

| PIXEL JOINING UNIT |

374

| SEGMENT EXTENSION UNIT |

323

# FIG.19

```
START OF PROCESSING TO
EXTRACT DEBRIS BOUNDARIES

        CONVERT PIXEL LINE          S151
        INTO THICK LINES

        CONVERT THICKENED LINES     S152
        INTO THIN LINES

            JOIN PIXELS             S153

          EXTEND SEGMENT            S154

        CONVERT EACH DEBRIS         S155
        BOUNDARY INTO THICK LINE

            RETURN
```

# FIG.20

402    401

EP 1 873 514 A1

FIG.21

# FIG.22

# FIG.23

# F I G . 2 4

# FIG.25

FLUORESCENCE
INTENSITY

351D

352B

352D

D

VECTOR-DIRECTION
COORDINATE

EP 1 873 514 A1

# FIG.26

441 SPOT DIVISION UNIT → 442 INFORMATION COMPUTATION UNIT → 443 AREA SELECTION UNIT → 444 OUTPUT UNIT

85

EP 1 873 514 A1

# FIG.27

START OF PROCESSING TO COMPUTE
HYBRIDIZATION LEVEL AND RELIABILITY

DIVIDE SPOT AREA INTO
SPOT INTERNAL AREAS    S201

COMPUTE HYBRIDIZATION
VALUE AND RELIABILITY FOR    S202
EACH SPOT INTERNAL AREA

SELECT SPOT INTERNAL AREA    S203

OUTPUT HYBRIDIZATION    S204
VALUE AND RELIABILITY
FOR EACH SPOT

RETURN

# FIG.28

# FIG.29

EP 1 873 514 A1

# FIG.30

FIG.31

FLUORESCENCE INTENSITY
(MEASUREMENT 2)

$CONFIDENCE_e(pf) = w_c \times w_{sat} \times VARIANCE(pf)^{-1}$

FLUORESCENCE INTENSITY
(MEASUREMENT 1)

EP 1 873 514 A1

# FIG.32

721                  722                  723

| RELIABILITY COMPUTATION UNIT | → | RELIABILITY DETERMINATION UNIT | → | FLAG SETTING UNIT |

443

# FIG.33

START OF PROCESSING TO SELECT
SPOT INTERNAL AREA

COMPUTE
(RELIABILITY OF SPOT INTERNAL
AREA × SPOT RELIABILITY) — S251

CORRECTED
SPOT INTERNAL AREA
RELIABILITY < THRESHOLD
VALUE? — S252 — TRUE

FAULT

USED IN
RELIABILITY DETERMINATION
USING MECHANICAL
LEARNING? — S253 — TRUE

FAULT

SET SCRAPPING FLAG BASED
ON RELIABILITY OF DATA OF
SPOT INTERNAL AREA — S254

RETURN

FIG.34

# FIG.35

```
    751                  752                  753                 754
┌─────────────────┐  ┌─────────────────┐  ┌──────────────┐  ┌─────────────────┐
│SPOT-COUNT       │  │COMBINATION      │  │COMPUTATION   │  │COMBINATION      │
│DETERMINATION    │─▶│GENERATION       │─▶│UNIT          │─▶│SELECTION        │
│UNIT             │  │UNIT             │  │              │  │UNIT             │
└─────────────────┘  └─────────────────┘  └──────────────┘  └─────────────────┘
         │
         │                755
         │           ┌─────────────────┐
         │           │FLAG SETTING     │
         └──────────▶│UNIT             │
                     └─────────────────┘

                              444
```

EP 1 873 514 A1

# FIG.36

START OF PROCESSING TO OUTPUT HYBRIDIZATION
VALUE AND RELIABILITY FOR EACH SPOT

S301
NUMBER OF DIVIDED
SPOTS < THRESHOLD VALUE?

FAULT

SET SCRAPPING FLAG
BASED ON RELIABILITY  S305

TRUE

GENERATE COMBINATIONS
OF SPOT INTERNAL AREAS  S302

COMPUTE OUTPUT HYBRIDIZATION LEVEL
AND RELIABILITY FOR EACH SPOT  S303

SELECT COMBINATION PROVIDING HIGHEST
RELIABILITY (SMALLEST VARIANCE)  S304

RETURN

EP 1 873 514 A1

# FIG.37

EP 1 873 514 A1

# FIG.38

# FIG.39

801

| $N$ | | | $n$ |
|---|---|---|---|
| $sh_1$ | $Sr_1$ | $f_1$ | $P_1$ |
| $sh_2$ | $Sr_2$ | $f_2$ | $P_2$ |
| $sh_3$ | $Sr_3$ | $f_3$ | $P_3$ |
| ⋮ | ⋮ | ⋮ | ⋮ |
| $sh_m$ | $Sr_m$ | $f_m$ | $P_m$ |

802

| $N$ | $P_{11}$ | $P_{12}$ | $P_{13}$ | $\cdots$ | $P_{1N}$ |
|---|---|---|---|---|---|

803

| $an$ | selected_no | | |
|---|---|---|---|
| SPOT RELIABILITY | | | |
| $ah_1^{1}$ | $ar_1^{1}$ | $pn_1^{1}$ | $f_1^{1}$ |
| $ah_1^{2}$ | $ar_1^{2}$ | $pn_1^{2}$ | $f_1^{2}$ |
| $ah_1^{3}$ | $ar_1^{3}$ | $pn_1^{3}$ | $f_1^{3}$ |
| ⋮ | ⋮ | ⋮ | ⋮ |

EP 1 873 514 A1

# FIG.40

EP 1 873 514 A1

# FIG.41

SPOT ELIMINATION
PATTERN DB

SVM

EP 1 873 514 A1

# FIG.42

EP 1 873 514 A1

## INTERNATIONAL SEARCH REPORT

| International application No. |
| --- |
| PCT/JP2006/307743 |

**A. CLASSIFICATION OF SUBJECT MATTER**
*G01N21/64*(2006.01)

According to International Patent Classification (IPC) or to both national classification and IPC

**B. FIELDS SEARCHED**

Minimum documentation searched (classification system followed by classification symbols)
G01N21/00-G01N21/01, G01N21/17-G01N21/83, G01N33/48-G01N33/98

Documentation searched other than minimum documentation to the extent that such documents are included in the fields searched
Jitsuyo Shinan Koho        1922-1996    Jitsuyo Shinan Toroku Koho    1996-2006
Kokai Jitsuyo Shinan Koho   1971-2006    Toroku Jitsuyo Shinan Koho    1994-2006

Electronic data base consulted during the international search (name of data base and, where practicable, search terms used)
JSTPlus(JDream2)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2004-340574 A  (Toagosei Co., Ltd.),<br>02 December, 2004 (02.12.04),<br>Par. No. [0003]<br>& US 2004/201840 A1    & EP 1422514 A1<br>& WO 2003/012411 A1 | 1,6,9-11 |
| Y | WO 2004/015418 A1  (SMARTBEAD TECHNOLOGIES LTD.),<br>19 February, 2004 (19.02.04),<br>Page 18, line 3 to page 22, line 27<br>& JP 2005-535874 A    & EP 1529214 A<br>& GB 2391867 A | 1,6,9-11 |
| Y | JP 2004-340738 A  (Sysmex Corp.),<br>02 December, 2004 (02.12.04),<br>Full text<br>(Family: none) | 1,6,9-11 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 June, 2006 (15.06.06) | 27 June, 2006 (27.06.06) |

| Name and mailing address of the ISA/<br>    Japanese Patent Office | Authorized officer |
| --- | --- |
| Facsimile No. | Telephone No. |

Form PCT/ISA/210 (second sheet) (April 2005)

**EP 1 873 514 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002257730 A